# EUROPEAN PATENT APPLICATION

(11) **EP 4 116 419 A1**
(43) Date of publication of application: **11.01.2023**
(21) Application number: 21764552.2
(22) Date of filing: 04.03.2021
(51) Int. Cl.: C12N 15/113, A61K 31/712, A61K 31/7125, A61K 47/54, A61K 47/55, A61K 47/64, A61K 48/00, A61P 9/06, A61P 43/00, C07H 21/04

(54) **ANTISENSE OLIGONUCLEOTIDE OF CALM2**

(30) Priority: 04.03.2020 JP 2020036715; 18.08.2020 JP 2020138019
(71) Applicant: Nissan Chemical Corporation, Tokyo 103-6119 (JP); Kyoto University, Kyoto-shi, Kyoto 606-8501 (JP)
(72) Inventor: IRIYAMA Yusuke, Funabashi-shi, Chiba 274-8507 (JP); KONDO Yoshiki, Shiraoka-shi, Saitama 349-0294 (JP); HIDAKA Yuuki, Shiraoka-shi, Saitama 349-0294 (JP); ISHIKAWA Ryutarou, Shiraoka-shi, Saitama 349-0294 (JP); TSUKADA Hiroyuki, Funabashi-shi, Chiba 274-8507 (JP); MAKIYAMA Takeru, Kyoto-shi, Kyoto 606-8501 (JP); YAMAMOTO Yuta, Kyoto-shi, Kyoto 606-8501 (JP)
(74) Representative: dompatent von Kreisler Selting Werner - Partnerschaft von Patent- und Rechtsanwälten mbB
(86) International application number: PCT/JP2021/008514
(87) International publication number: WO 2021/177418

(57) **Abstract**

The present invention provides a compound or a pharmaceutically acceptable salt thereof containing a modified oligonucleotide with a length of 8 to 80 consecutive nucleosides, in which the modified oligonucleotide has a nucleobase sequence containing at least 8 consecutive nucleobases contained in a nucleobase sequence of any one of SEQ ID NOs: 3 to 73. With the compound or a pharmaceutically acceptable salt thereof, it is possible to treat a disease or a condition against which inhibition of CALM2 gene expression by controlling of the CALM2 gene expression is effective (particularly, congenital long QT syndrome).

## Description

### Technical Field

The present application relates to an antisense oligonucleotide of CALM2.

### Background Art

Congenital long QT syndrome (congenital LQTS) is a fatal congenital arrhythmia disease in which a QT interval on the electrocardiogram is prolonged, leading to fainting or sudden death due to ventricular arrhythmias (Non Patent Literature 1). The causes of the disease have been mostly reported to be mutations in genes for ion channels or regulatory proteins thereof expressed in the heart.

As the genes responsible for the disease, KCNQ1 and KCNH2 associated with potassium channels and SCN5A associated with sodium channels have been known so far, however, calmodulin genes (CALM1 to CALM3) have been recently identified.

Calmodulin is a ubiquitously expressed calcium-sensing protein, and three different genes (CALM1 to CALM3) encode calmodulin proteins having the same amino acid sequence. Calmodulin regulates various proteins including multiple ion channels. In particular, calmodulin regulates the inactivation of L-type calcium channels (LTCC) expressed in the heart (Non Patent Literature 3).

In many of patients with congenital LQTS caused by the calmodulin gene, the disease is severe, and many do not survive into adulthood due to sudden death caused in childhood. It has been reported that a hetero-missense mutation in the CALM gene is associated with the occurrence of the severe arrhythmia (Non Patent Literature 2). It is considered that a missense mutation in one of the three different CALM genes exhibits a dominant negative activity, resulting in a serious symptom (Non Patent Literature 4).

Research has also been conducted on a genome-editing technology targeting CALM2. Non-mutant allele-specific knockdown using the CRISPRi system (Non Patent Literature 6) and mutant allele-specific knockout using the CRISPR/Cas9 system (Non Patent Literature 4) have been reported. The mutant allele-specific knockout by the CRISPR/Cas9 system has been shown to be a possible gene therapy method effective for patients with congenital LQTS caused by the dominant negative activity of the CALM gene (Non Patent Literature 4).

### Citation List

### Non Patent Literature

Non Patent Literature 1: Guideline for Treatment of Hereditary Arrhythmia (2017 revised edition), pages 8-19
Non Patent Literature 2: Heart Rhythm, 2015; 12: 423-424
Non Patent Literature 3: Heart Rhythm, 2016; 13: 2012-2019
Non Patent Literature 4: Human Molecular Genetics, 2017; 26: 1670-1677
Non Patent Literature 5: European Heart Journal, 2019;40: 2964-2975
Non Patent Literature 6: Circulation Research, 2017; 120: 39-48

### Summary of Invention

### Technical Problem

As a treatment method for congenital LQTS, for example, treatment in which an implantable cardioverter defibrillator is used order to prevent sudden death has been considered, however, the method is problematic, since it is a symptomatic therapy, psychological and physical burden of the treatment is great on the patients many of whom are children, and invasion and complications occur after implantation (Non Patent Literature 5).

Moreover, there are no examples of clinical application of a method using any of the CRISPRi system and CRISPR/Cas9 system, and the clinical application is known to involve many problems.

Therefore, establishment of drug treatment for congenital LQTS that can overcome the problems of conventional surgical therapy or gene therapy is demanded. In particular, it is considered that the need for a novel therapeutic agent which can prevent a fatal arrhythmic event and improve prognosis is extremely high, and clinical implication thereof is also great (Non Patent Literature 5).

At present, there are many cases in which a β-blocker is administered in LQT15 cases caused by a CALM2 mutation, however, the treatment effect thereof has been reported to be low (Non Patent Literature 5).

Thus, a drug that is useful for treating, preventing, or ameliorating congenital LQTS is still needed to be provided.

Hence, an object of the present application is to provide a novel compound or a pharmaceutically acceptable salt thereof inhibiting expression of the CALM2 gene that is known to be associated with congenital LQTS.

### Solution to Problem

After diligent study in view of the above problems, the present inventors found that a compound containing a modified oligonucleotide having a specific sequence or a pharmaceutically acceptable salt thereof has an excellent inhibitory action on the CALM2 gene expression.

That is, the present application provides, in summary, the following.
1.
   A compound or a pharmaceutically acceptable salt thereof containing a modified oligonucleotide with a length of 8 to 80 consecutive nucleosides, in which the modified oligonucleotide has a nucleobase sequence containing at least 8 consecutive nucleobases contained in a nucleobase sequence of any one of SEQ ID NOs: 3 to 73 (3 to 20 in one embodiment).
2.
   The compound or a pharmaceutically acceptable salt thereof according to 1., in which the modified oligonucleotide has a nucleobase sequence containing a nucleobase sequence of any one of SEQ ID NOs: 3 to 73 (3 to 20 in one embodiment).
3.
   The compound or a pharmaceutically acceptable salt thereof according to 1. or 2., in which the modified oligonucleotide has a nucleobase sequence consisting of a nucleobase sequence of any one of SEQ ID NOs: 3 to 73 (3 to 20 in one embodiment).
4.
   A compound or a pharmaceutically acceptable salt thereof containing a modified oligonucleotide with a length of 8 to 80 consecutive nucleosides which is complementary to at least a part of a nucleobase sequence selected from the group consisting of the nucleobase sequences indicated by nucleobase position nos. 102 to 117, 160 to 175, 183 to 205, 212 to 227, 322 to 337, 365 to 405, 411 to 434, 464 to 494, 506 to 521, 606 to 635, 636 to 651, 692 to 707, 715 to 749, 754 to 801, 829 to 857, 862 to 934, 951 to 970, 995 to 1010, 1006 to 1021, 1036 to 1051, 1062 to 1077, 1081 to 1104, 1138 to 1166, 1188 to 1203, and 1239 to 1254 in SEQ ID NO: 1, in which the modified oligonucleotide has at least 80% complementarity to at least a part of the nucleobase sequence in SEQ ID NO: 1.
5.
   A compound or a pharmaceutically acceptable salt thereof containing a modified oligonucleotide with a length of 8 to 80 consecutive nucleosides which is complementary to at least a part of a nucleobase sequence selected from the group consisting of the nucleobase sequences indicated by nucleobase position nos. 102 to 117, 160 to 175, 183 to 198, 212 to 227, 322 to 337, 365 to 380, 387 to 402, 479 to 494, 620 to 635, 715 to 730, 862 to 877, 896 to 934, 995 to 1010, 1062 to 1077, 1089 to 1104, 1140 to 1166, and 1239 to 1254 in SEQ ID NO: 1, in which the modified oligonucleotide has at least 80% complementarity to at least a part of the nucleobase sequence in SEQ ID NO: 1.
6.
   A compound or a pharmaceutically acceptable salt thereof containing a modified oligonucleotide with a length of 8 to 80 consecutive nucleosides which is complementary to at least a part of the nucleobase sequence indicated by nucleobase position nos. 896 to 934, preferably, 907 to 922, in SEQ ID NO: 1, in which the modified oligonucleotide has at least 80% complementarity to at least a part of the nucleobase sequence in SEQ ID NO: 1.
7.
   A compound or a pharmaceutically acceptable salt thereof containing a modified oligonucleotide with a length of 8 to 80 consecutive nucleosides which is complementary to at least a part of a nucleobase sequence selected from the group consisting of the nucleobase sequences indicated by nucleobase position nos. 102 to 117, 160 to 175, 1624 to 1639, 1661 to 1676, 3355 to 3370, 5859 to 5881, 13959 to 13974, 14069 to 14084, 14228 to 14278, 14284 to 14307, 14764 to 14794, 14806 to 14821, 15824 to 15869, 15910 to 15925, 15933 to 15967, 15972 to 16019, 16047 to 16075, 16080 to 16152, 16169 to 16188, 16213 to 16239, 16254 to 16269, 16280 to 16295, 16299 to 16322, 16356 to 16384, 16406 to 16421, and 16457 to 16472 in SEQ ID NO: 2, in which the modified oligonucleotide has at least 80% complementarity to at least a part of the nucleobase sequence in SEQ ID NO: 2.
8.
   A compound or a pharmaceutically acceptable salt thereof containing a modified oligonucleotide with a length of 8 to 80 consecutive nucleosides which is complementary to at least a part of a nucleobase sequence selected from the group consisting of the nucleobase sequences indicated by nucleobase position nos. 102 to 117, 160 to 175, 5859 to 5874, 13959 to 13974, 14069 to 14084, 14238 to 14253, 14260 to 14275, 14779 to 14794, 15838 to 15853, 15933 to 15948, 16080 to 16095, 16114 to 16152, 16213 to 16228, 16280 to 16295, 16307 to 16322, 16358 to 16384, and 16457 to 16472 in SEQ ID NO: 2, in which the modified oligonucleotide has at least 80% complementarity to at least a part of the nucleobase sequence in SEQ ID NO: 2.
9.
   A compound or a pharmaceutically acceptable salt thereof containing a modified oligonucleotide with a length of 8 to 80 consecutive nucleosides which is complementary to at least a part of the nucleobase sequence indicated by nucleobase position nos. 16114 to 16152, preferably, 16125 to 16140, in SEQ ID NO: 2, in which the modified oligonucleotide has at least 80% complementarity to at least a part of the nucleobase sequence in SEQ ID NO: 2.
10.
   The compound or a pharmaceutically acceptable salt thereof according to any one of 1. to 9., in which the modified oligonucleotide contains a phosphorothioate bond.
11.
   The compound or a pharmaceutically acceptable salt thereof according to any one of 1. to 10., in which the modified oligonucleotide contains at least one selected from the group consisting of a 2'-modified nucleoside and a 2'-4'-bridged nucleoside.
12.
   The compound or a pharmaceutically acceptable salt thereof according to 11., in which the 2'-4'-bridged nucleoside is at least one selected from the group consisting of an LNA, an ENA, a cEt, a BNA^{NC}, an AmNA, an scpBNA, and a GuNA.
13.
   The compound or a pharmaceutically acceptable salt thereof according to 12., in which the 2'-4'-bridged nucleoside is an LNA.
14.
   The compound or a pharmaceutically acceptable salt thereof according to any one of 11. to 13., in which the 2'-modified nucleoside is at least one selected from the group consisting of a 2'-O-MCE nucleoside, a 2'-O-MOE nucleoside, a 2'-O-NMA nucleoside, and a 2'-O-Me nucleoside.
15.
   The compound or a pharmaceutically acceptable salt thereof according to 14., in which the 2'-modified nucleoside is at least one selected from the group consisting of a 2'-O-MCE nucleoside and a 2'-O-MOE nucleoside.
16.
   The compound or a pharmaceutically acceptable salt thereof according to any one of 1. to 15., in which the modified oligonucleotide contains 5-methylcytosine.
17.
   The compound or a pharmaceutically acceptable salt thereof according to any one of 1. to 16.,
   in which the modified oligonucleotide includes a gap segment, a 5' wing segment, and a 3' wing segment,
   the gap segment contains at least 2 deoxyribonucleosides, and the 5' terminal and the 3' terminal of the gap segment are deoxyribonucleosides,
   the nucleoside at the 3' terminal of the 5' wing segment is a sugar-modified nucleoside and is linked to the 5' terminal of the gap segment, and
   the nucleoside at the 5' terminal of the 3' wing segment is a sugar-modified nucleoside and is linked to the 3' terminal of the gap segment.
18. The compound or a pharmaceutically acceptable salt thereof according to 17.,
   in which the gap segment consists of 5 to 30 deoxyribonucleosides,
   the 5' wing segment and the 3' wing segment each independently consist of 1 to 10 sugar-modified nucleosides independently selected from the group consisting of an LNA, a 2'-O-MCE nucleoside, and a 2'-O-MOE nucleoside, each of the wing segments containing at least one phosphorothioate bond, and
   each cytosine in the gap segment and each wing segments is replaced with 5-methylcytosine.
19. The compound or a pharmaceutically acceptable salt thereof according to 18.,
   in which the gap segment consists of 8 to 12 deoxyribonucleosides,
   the 5' wing segment and the 3' wing segment each independently consist of 2 to 5 sugar-modified nucleosides independently selected from the group consisting of an LNA and a 2'-O-MCE nucleoside, and
   the gap segment contains at least one phosphorothioate bond.
20. The compound according to 19., in which the 5' wing segment and the 3' wing segment are each independently selected from the group consisting of LL, LLL, VLL, LVL, LLV, LVV, VLV, VVL, VVLL, VLVL, LVLV, LLVV, VLLL, LVLL, LLVL, LLLV, LVVV, VLVV, VVLV, and VVVL, where L represents an LNA, and V represents a 2'-O-MCE nucleoside.
21. The compound or a pharmaceutically acceptable salt thereof according to any one of 1. to 20., in which the modified oligonucleotide is 11 to 50, preferably, 15 to 25 consecutive nucleosides long.
22. The compound or a pharmaceutically acceptable salt thereof according to any one of 1. to 21., in which the modified oligonucleotide is an antisense oligonucleotide.
23. The compound or a pharmaceutically acceptable salt thereof according to any one of 1. to 22., in which the compound containing the modified oligonucleotide contains a prodrug moiety.
24.
   The compound or a pharmaceutically acceptable salt thereof according to any one of 1. to 23., in which the compound containing the modified oligonucleotide contains a functional molecule.
25.
   The compound or a pharmaceutically acceptable salt thereof according to 24., in which the functional molecule is a group derived from a molecule having a function of delivering the modified oligonucleotide to a target site.
26.
   The compound or a pharmaceutically acceptable salt thereof according to 24. or 25., in which the functional molecule is selected from the group consisting of a sugar, a lipid, a peptide, a protein, and a derivative thereof.
27.
   The compound or a pharmaceutically acceptable salt thereof according to any one of 24. to 26., in which the functional molecule is a lipid selected from the group consisting of cholesterol, a vitamin, a steroid, a C5-30 saturated fatty acid, and a C5-30 unsaturated fatty acid.
28.
   The compound or a pharmaceutically acceptable salt thereof according to any one of 1. to 23., in which the compound consists of the modified oligonucleotide.
29.
   The salt according to any one of 1. to 28., in which the pharmaceutically acceptable salt is a sodium salt.
30.
   A drug comprising the compound or a pharmaceutically acceptable salt according to any one of 1. to 29..
31.
   A drug for treating, preventing, and/or ameliorating a disease or a condition against which an inhibitory action on CALM2 gene expression is effective, the drug comprising the compound or a pharmaceutically acceptable salt according to any one of 1. to 29..
32.
   An inhibitor of CALM2 gene expression comprising the compound or a pharmaceutically acceptable salt according to any one of 1. to 29..
33.
   A drug for treating, preventing, and/or ameliorating congenital long QT syndrome, the drug comprising the compound or a pharmaceutically acceptable salt according to any one of 1. to 29..
34.
   A drug for treating, preventing, and/or ameliorating calmodulinopathy, the drug comprising the compound or a pharmaceutically acceptable salt according to any one of 1. to 29..
35.
   A method for treating, preventing, and/or ameliorating a disease or a condition against which an inhibitory action on CALM2 gene expression is effective, the method comprising a step of administering an effective dose of the compound or a pharmaceutically acceptable salt according to any one of 1. to 29. to a subject in need of the compound or a pharmaceutically acceptable salt.
36.
   A method for inhibiting CALM2 gene expression, the method comprising a step of administering an effective dose of the compound or a pharmaceutically acceptable salt according to any one of 1. to 29. to a subject in need of the compound or a pharmaceutically acceptable salt.
37.
   A method for treating, preventing, and/or ameliorating congenital long QT syndrome, the method comprising a step of administering an effective dose of the compound or a pharmaceutically acceptable salt according to any one of 1. to 29. to a subject in need of the compound or a pharmaceutically acceptable salt.
38.
   A method for treating, preventing, and/or ameliorating calmodulinopathy, the method comprising a step of administering an effective dose of the compound or a pharmaceutically acceptable salt according to any one of 1. to 29. to a subject in need of the compound or a pharmaceutically acceptable salt.
39.
   The compound according to any one of 1. to 29., which is used as a drug.
40.
   The compound according to any one of 1. to 29., which is used for treating, preventing, and/or ameliorating a disease or a condition against which an inhibitory action on CALM2 gene expression is effective.
41.
   The compound according to any one of 1. to 29., which is used for inhibiting CALM2 gene expression.
42.
   The compound according to any one of 1. to 29., which is used for treating, preventing, and/or ameliorating congenital long QT syndrome.
43.
   The compound according to any one of 1. to 29., which is used for treating, preventing, and/or ameliorating calmodulinopathy.
44.
   Use of the compound or a pharmaceutically acceptable salt according to any one of 1. to 29. in production of a drug for treating, preventing, and/or ameliorating a disease or a condition against which an inhibitory action on CALM2 gene expression is effective.
45.
   Use of the compound or a pharmaceutically acceptable salt according to any one of 1. to 29. in production of an inhibitor of CALM2 gene expression.
46.
   Use of the compound or a pharmaceutically acceptable salt according to any one of 1. to 29. in production of a drug for treating, preventing, and/or ameliorating congenital long QT syndrome.
47.
   Use of the compound or a pharmaceutically acceptable salt according to any one of 1. to 29. in production of a drug for treating, preventing, and/or ameliorating calmodulinopathy.

### Advantageous Effects of Invention

A novel compound containing a modified oligonucleotide or a pharmaceutically acceptable salt thereof is provided. Since the compound or a pharmaceutically acceptable salt thereof has an excellent inhibitory action on the CALM2 gene expression, it can be particularly used for treating, ameliorating, and/or preventing congenital long QT syndrome and even calmodulinopathy.

### Brief Description of Drawings

Fig. 1 is a graph showing effects of the ASO according to the present embodiment on expression levels of CALM1, CALM2, and CALM3 in human hepatoma-derived cells.
Fig. 2 shows results of measuring action potentials in cardiomyocytes differentiated from LQT15 patient-derived human iPS cells in the presence and absence of the ASO according to the present embodiment.
Fig. 3 is a graph showing an effect of the ASO according to the present embodiment on an action potential duration in cardiomyocytes differentiated from LQT15 patient-derived human iPS cells.
Fig. 4 is a graph showing an effect of the ASO according to the present embodiment on the expression level of CALM2 in cardiomyocytes differentiated from LQT15 patient-derived human iPS cells.
Fig. 5 is a graph showing effects of ASOs according to the present embodiment on the expression level of CALM2 in hearts of C57BL/6J mice administered with the ASOs.
Fig. 6 is a graph showing effects of the ASOs according to the present embodiment on the expression level of CALM2 in livers of C57BL/6J mice administered with the ASOs.
Fig. 7 is a graph showing effects of the ASOs according to the present embodiment on the expression level of CALM2 in kidneys of C57BL/6J mice administered with the ASOs.
Fig. 8 is a graph showing effects of ASOs according to the present embodiment on the expression level of CALM2 in hearts of C57BL/6J mice administered with the ASOs.
Fig. 9 is a graph showing effects of ASOs according to the present embodiment on the expression level of CALM2 in hearts of C57BL/6J mice administered with the ASOs.
Fig. 10 is a graph showing effects of ASOs according to the present embodiment on the expression level of CALM2 in hearts of C57BL/6J mice administered with the ASOs.
Fig. 11 is a graph showing effects of the ASOs according to the present embodiment on the expression level of CALM2 in livers of C57BL/6J mice administered with the ASOs.
Fig. 12 is a graph showing effects of the ASOs according to the present embodiment on the expression level of CALM2 in kidneys of C57BL/6J mice administered with the ASOs.

### Description of Embodiments

It is to be understood that both the summary described above and the detailed descriptions below are merely exemplary and descriptive and do not limit the claimed inventions in any manner. In the present specification, items indicated in singular forms connote examples of plural forms, unless interpreting the items as the plural forms causes clear divergence from the context or otherwise specifically described.

Furthermore, in the present specification, an antisense oligonucleotide may be referred to as an "ASO".

Nucleic acids in nature are most basically composed of adenosine (A), thymidine (T) (or uridine (U)), cytidine (C), and guanosine (G). These basic nucleic acids are often referred to as AT(U)GC and the like. Therefore, in the present specification, regarding a sequence of, for example, the CALM2 gene or the like, when the sequence is indicated by a "nucleobase sequence" or "SEQ ID NO", it is basically a sequence composed of A, G, C, T, and U.

On the other hand, a nucleic acid constituting the ASO in the present application includes not only the basic nucleic acids (AT(U)CG) but also a nucleic acid resulting from structural modification thereof. Although details of the modification will be described later, the modification includes modification of a sugar moiety, a internucleoside bond, and/or a nucleobase.

Thus, in the present specification, in a case where a nucleobase sequence of, for example, the ASO in the present application or the like is indicated using A, G, C, T, and U in descriptions of a "compound" or a "compound designated a compound number (a P number)", A, G, C, T, and U also include structurally modified A, G, C, T, and U.

More detailed description is as follows.

Unless otherwise indicated, the following terms have the following meanings.

"May be substituted" means being unsubstituted or substituted.

A "nucleoside" is a term well-known to those skilled in the art, and is generally understood as a molecule in which a sugar and a nucleobase are bonded and which can be a unit constituting a nucleic acid. In the present specification, the concept of a nucleoside is broader and includes a deoxyribonucleoside, a ribonucleoside, and a sugar-modified nucleoside described later. The nucleobase may be modified.

A "deoxyribonucleoside" refers to a molecule having a nucleobase at the carbon atom at the position 1' in 2'-deoxyribose. The deoxyribonucleoside in the present application may be a naturally occurring deoxyribonucleoside or a deoxyribonucleoside in which a nucleobase moiety of a naturally occurring deoxyribonucleoside is modified. A plurality of modifications may be subjected to one deoxyribonucleoside in combination. The modified deoxyribonucleoside is described in, for example, Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A, and the like.

A "ribonucleoside" refers to a molecule having a nucleobase at the carbon atom at the position 1' in ribose. The ribonucleoside in the present application may be a naturally occurring ribonucleoside or a ribonucleoside in which a nucleobase moiety of a naturally occurring ribonucleoside is modified. A plurality of modifications may be subjected to one ribonucleoside in combination. The modified ribonucleoside is described in, for example, Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A, and the like.

A "modified sugar" refers to
(Z1) a molecule in which ribose or 2'-deoxyribose is partially substituted with one or more substituents,
(Z2) a pentasaccharide or a hexasaccharide different from ribose and 2'-deoxyribose (for example, hexitol, threose, or the like),
(Z3) a molecule in which the entire ribose or 2'-deoxyribose or a tetrahydrofuran ring thereof is replaced with a 5- to 7-membered saturated or unsaturated ring (for example, cyclohexane, cyclohexene, morpholine, or the like) or a partial structure in which a 5- to 7-membered ring can be formed by a hydrogen bond (for example, a peptide structure), or
(Z4) a molecule in which ribose or 2'-deoxyribose is replaced with an alkylene glycol having 2 to 6 carbon atoms (for example, ethylene glycol, propylene glycol, or the like).

The modified sugar includes a "2'-modified sugar" and a "2'-4'-bridged sugar" described later.

Examples of the modified sugar and a sugar-modified nucleoside described later include the sugars, sugar-modified nucleosides, and the like disclosed in JP H10-304889 A, WO 2005/021570 A, JP H10-195098 A, JP 2002-521310 A, WO 2007/143315 A, WO 2008/043753 A, WO 2008/029619 A, WO 2008/049085 A, WO 2017/142054 A, and the like (hereinafter, these literatures are referred to as a "literature on an antisense method") as being preferably used for the antisense method. In addition, Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A, and the like also disclose modified sugars and sugar-modified nucleosides.

Examples of the modified sugar partially substituted with one substituent include ribose or 2'-deoxyribose in which an arbitrary position in the sugar moiety is substituted with the following (i) or (ii).
(i) A C₁₋₆ alkyl group
(ii) A C₁₋₆ alkyl group substituted with at least one selected from the group consisting of a halogen atom, a C₁₋₆ alkoxy group, a halo C₁₋₆ alkoxy group, a mono- or di-C₁₋₆ alkylamino group, a 5- to 10-membered heterocyclic group, a carboxy group, a carbamoyl group, and a N-substituted carbamoyl group.

Here, examples of the N-substituted carbamoyl group include a N-methyl-carbamoyl group and a N-ethyl-carbamoyl group, where the methyl group and the ethyl group of the N-methyl-carbamoyl group and the N-ethyl-carbamoyl group may be substituted with a 5- to 10-membered heterocyclic group or a mono- or di-C₁₋₆ alkylamino group. Specific examples of the N-substituted carbamoyl group include a N-methylcarbamoyl group, a N-ethylcarbamoyl group, a N-dimethylaminoethylcarbamoyl group, a N-morpholinoethylcarbamoyl group, a N-(2-pyridylethyl)carbamoyl group, a N-((benzimidazol-1-yl)ethyl)carbamoyl group, and the like.

The "sugar-modified nucleoside" refers to a molecule having the "modified sugar" in place of the sugar moiety of a deoxyribonucleoside or a ribonucleoside. The "sugar-modified nucleoside" includes, for example, a "2'-modified nucleoside" and a "2'-4'-bridged nucleoside" described later. In a case where the modified sugar satisfies the above definition of (Z3), the sugar-modified nucleoside also includes a molecule in which the modified sugar and the nucleobase are bonded through a methylene chain or the like.

A "2'-modified sugar" refers to a non-bridged sugar in which the oxygen atom or carbon atom at the position 2' in ribose is modified, and includes "2'-O-Me", "2'-O-MOE", "2'-O-MCE", "2'-O-NMA", "2'-DMAECE", "2'-MorECE", "2'-PyECE", and "BimECE".

A "2'-modified nucleoside" refers to a molecule having a nucleobase at the position 1' in the 2'-modified sugar, and examples thereof include a "2'-O-Me nucleoside", a "2'-O-MOE nucleoside", a "2'-O-MCE nucleoside", a "2'-O-NMA nucleoside", a "2'-DMAECE nucleoside", a "2'-MorECE nucleoside", a "2'-PyECE nucleoside", and a "BimECE nucleoside".

"2'-O-Me" (also referred to as 2'-O-methyl) represents a sugar in which the hydroxy group at the position 2' in ribose is replaced with a methoxy group.

The "2'-O-Me nucleoside" (also referred to as a 2'-O-methyl nucleoside) refers to a molecule having a nucleobase at the position 1' in "2'-O-Me".

"2'-O-MOE" (also referred to as 2'-O-methoxyethyl) represents a sugar in which the hydroxy group at the position 2' in ribose is replaced with a 2-methoxyethyloxy group.

The "2'-O-MOE nucleoside" (also referred to as a 2'-O-methoxyethyl nucleoside) refers to a molecule having a nucleobase at the position 1' in "2'-O-MOE".

"2'-O-MCE" (also referred to as 2'-O-methylcarbamoylethyl) represents a sugar in which the hydroxy group at the position 2' in ribose is replaced with a methylcarbamoylethyloxy group.

The "2'-O-MCE nucleoside" (also referred to as a 2'-O-methylcarbamoylethyl nucleoside) refers to a molecule having a nucleobase at the position 1' in "2'-O-MCE".

"2'-O-NMA" represents a sugar in which the hydroxy group at the position 2' in ribose is replaced with a 2-[(methylamino)-2-oxoethyl]oxy group.

The "2'-O-NMA nucleoside" refers to a molecule having a nucleobase at the position 1' in "2'-O-NMA".

"2'-O-AP" represents a sugar in which the hydroxy group at the position 2' in ribose is replaced with a 3-aminopropyloxy group.

A "2'-O-AP nucleoside" refers to a molecule having a nucleobase at the position 1' in "2'-O-AP".

"2'-F" represents a sugar in which the hydroxy group at the position 2' in ribose is replaced with a fluorine atom.

A "2'-F nucleoside" refers to a molecule having a nucleobase at the position 1' in "2'-F".

"2'-DMAECE" is a modified sugar having the following structure.

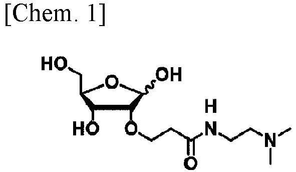

"2'-MorECE" is a modified sugar having the following structure.

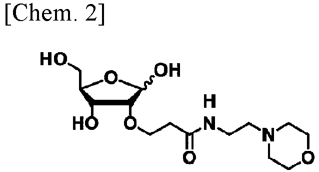

"2'-PyECE" is a modified sugar having the following structure.

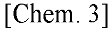

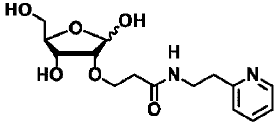

"BimECE" is a modified sugar having the following structure.

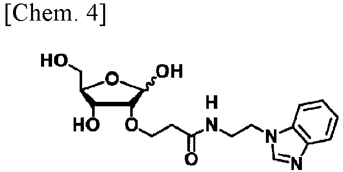

The "2'-DMAECE nucleoside", the "2'-MorECE nucleoside", the "2'-PyECE nucleoside", and the "BimECE nucleoside" refer to molecules having nucleobases at the position 1' in "2'-DMAECE", "2'-MorECE", "2'-PyECE", and "BimECE", respectively.

A "2'-4'-bridged sugar" refers to a sugar substituted with a bridging unit by a substitution at two positions, the positions 2' and 4', in ribose. Examples of the bridging unit include a C₂₋₆ alkylene group (the alkylene group is unsubstituted or substituted with one or more substituents selected from the group consisting of a halogen atom, an oxo group, and a thioxo group, and one or two methylene groups in the alkylene group are not replaced or independently replaced with a group selected from the group consisting of -O-, -NR¹- (R¹ represents a hydrogen atom, a C₁₋₆ alkyl group, or a halo C₁₋₆ alkyl group), and -S-).

A "2'-4'-bridged nucleoside" (2',4'-BNA) refers to a molecule having a nucleobase at the position 1' in the 2'-4'-bridged sugar. Examples of the "2'-4'-bridged nucleoside" include an α-L-methyleneoxy (4'-CH₂-O-2') BNA or a β-D-methyleneoxy (4'-CH₂-O-2') BNA which is also referred to as a LNA (locked Nucleic Acid (registered trademark)) described later, an ethyleneoxy (4'-(CH₂)₂-O-2') BNA which is also referred to as an ENA, a β-D-thio (4'-CH₂-S-2') BNA, an aminooxy (4'-CH₂-O-N(R¹¹)-2') BNA (R¹¹ is H or CH₃), an oxyamino (4'-CH₂-N(R¹²)-O-2') BNA (R¹² is H or CH₃) which is also referred to as a 2',4'-BNA^{NC}, a 2',4'-BNA^{COC}, a 3'-amino-2',4'-BNA, a 5'-methyl BNA, a (4'-CH(CH₃)-O-2') BNA which is also referred to as a cEt, a (4'-CH(CH₂OCH₃)-O-2') BNA which is also referred to as a cMOE-BNA, an amide-based BNA (4'-C(=O)-N(R¹³)-2') BNA (R¹³ is H or CH₃) which is also referred to as an AmNA, a (4'-C(spiro-cyclopropyl)-O-2') BNA which is also referred to as an scpBNA, a (4'-CH₂-N(R¹⁴)-2') BNA (R¹⁴ is C(=NH₂⁺)NHR¹⁵, and R¹⁵ is H or CH₃) which is also referred to as a GuNA, other BNAs known to those skilled in the art, and the like.

Though examples of the bond between a nucleobase and the carbon atom at the position 1' in the "deoxyribonucleoside", "ribonucleoside", "2'-modified nucleoside", "2'-4'-bridged nucleoside", and the like include an α-glycosidic bond and a β-glycosidic bond, the bond is generally a β-glycosidic bond. As the LNA, a β-D-methyleneoxy BNA is generally used.

"n-" stands for normal, "s-" stands for secondary, and "t-" stands for tertiary.

The "halogen atom" refers to a fluorine atom, a chlorine atom, a bromine atom, or an iodine atom.

The "C₁₋₆ alkyl group" refers to a linear or branched saturated hydrocarbon group having 1 to 6 carbon atoms, and examples thereof include a methyl group, an ethyl group, a n-propyl group, an isopropyl group, a n-butyl group, an isobutyl group, a s-butyl group, a t-butyl group, a n-pentyl group, an isopentyl group, a neopentyl group, a n-hexyl group, an isohexyl group, and the like.

The "halo C₁₋₆ alkyl group" refers to a group in which a hydrogen atom at an arbitrary position in the "C₁₋₆ alkyl group" is substituted with one or more "halogen atoms" described above.

A "C₁₋₆ alkylene group" refers to a divalent group in which one hydrogen atom at an arbitrary position is removed from a linear or branched saturated hydrocarbon group having 1 to 6 carbon atoms, and examples thereof include a methylene group, an ethylene (ethanediyl) group, a propane-1,3-diyl (trimethylene) group, a propane-2,2-diyl group, a 2,2-dimethyl-propane-1,3-diyl group, a hexane-1,6-diyl (hexamethylene) group, and a 3-methylbutane-1,2-diyl group.

The "C₂₋₆ alkylene group" refers to a linear or branched divalent group having 2 to 6 carbon atoms among the "C₁₋₆ alkylene groups" described above, and examples thereof are the same as those of the "C₁₋₆ alkylene group", except for the methylene group.

A "C₂₋₂₀ alkylene group" refers to a divalent group in which one hydrogen atom is removed from a linear or branched saturated hydrocarbon group having 2 to 20 carbon atoms at an arbitrary position. Similarly, a "C₈₋₁₂ alkylene group" refers to a divalent group in which one hydrogen atom at an arbitrary position is removed from a linear or branched saturated hydrocarbon group having 8 to 12 carbon atoms.

A "C₂₋₂₀ alkenylene group" refers to a divalent group in which one hydrogen atom is removed, at an arbitrary position, from a linear or branched unsaturated hydrocarbon group having 2 to 20 carbon atoms and at least one double bond.

The "C₁₋₆ alkoxy group" refers to a group in which the "C₁₋₆ alkyl group" is bonded to the oxy group.

The "halo C₁₋₆ alkoxy group" refers to a group in which a hydrogen atom at an arbitrary position in the "C₁₋₆ alkoxy group" is substituted with one or more "halogen atoms" described above.

The "mono- or di-C₁₋₆ alkylamino group" refers to a group in which one hydrogen atom in an amino group is replaced with one "C₁₋₆ alkyl group" or a group in which two hydrogen atoms in an amino group is replaced with two identical or different "C₁₋₆ alkyl groups", and examples thereof include methylamino, ethylamino, propylamino, isopropylamino, butylamino, dimethylamino, diethylamino, dipropylamino, dibutylamino, N-ethyl-N-methylamino groups, and the like.

The "5- to 10-membered heterocyclic group" refers to a 5- to 10-membered monocyclic or fused polycyclic aromatic or non-aromatic heterocyclic group having, in addition to carbon atoms, 1 to 4 heteroatoms selected from a nitrogen atom, a sulfur atom, and an oxygen atom as the ring-constituting atoms.

Preferable examples of the "5- to 10-membered heterocyclic group" include thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, triazolyl, tetrazolyl, triazinyl, benzothiophenyl, benzofuranyl, benzimidazolyl, benzoxazolyl, benzisoxazolyl, benzothiazolyl, benzoisothiazolyl, benzotriazolyl, imidazopyridinyl, thienopyridinyl, pyrrolopyridinyl, pyrazolopyridinyl, oxazolopyridinyl, thiazolopyridinyl, imidazopyrazinyl, imidazopyrimidinyl, aziridinyl, oxiranyl, azetidinyl, oxetanyl, thietanyl, tetrahydrothienyl, tetrahydrofuranyl, pyrrolinyl, pyrrolidinyl, oxopyrrolidinyl, imidazolinyl, oxoimidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, tetrahydropyridazinyl, dihydropyranyl, tetrahydropyranyl, tetrahydrothiopyranyl, morpholinyl, thiomorpholinyl, and the like.

The "oxo group" indicates a group substituted with an oxygen atom through a double bond (=O). In a case where a carbon atom is substituted with the oxo group, the oxo group forms a carbonyl group together with the carbon atom.

The "thioxo group" indicates a group substituted with a sulfur atom through a double bond (=S). In a case where a carbon atom is substituted with the thioxo group, the thioxo group forms a thiocarbonyl group together with the carbon atom.

The "nucleobase" is a purine base or a pyrimidine base and may be a naturally occurring nucleobase or a nucleobase obtained by modification of the naturally occurring nucleobase. Examples of the naturally occurring nucleobase include adenine (A), guanine (G), thymine (T), cytosine (C), and uracil (U). The "nucleobase" includes the naturally occurring nucleobase and a "modified nucleobase" described below.

Examples of the modification of the nucleobase in the "modified nucleobase" include halogenation, methylation, ethylation, n-propylation, isopropylation, cyclopropylation, n-butylation, isobutylation, s-butylation, t-butylation, cyclobutylation, hydroxylation, amination, thioation, demethylation, and the like. More specific examples of the modification include 5-methylation, 5-fluorination, 5-bromination, 5-iodization, and N4-methylation of cytosine; 2-thioation, 5-demethylation, 5-fluorination, 5-bromination, and 5-iodization of thymine; 2-thioation, 5-fluorination, 5-bromination, and 5-iodization of uracil; N6-methylation and 8-bromination of adenine; and N2-methylation 8-bromination of guanine, and the like. In addition, Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2018/155450 A and the like disclose examples of the modification of the nucleobase moiety in a nucleoside.

The nucleobase in a nucleoside is preferably at least one selected from the group consisting of adenine, guanine, thymine, cytosine, uracil, and 5-methylcytosine.

The "5-methylcytosine" refers to 5-methylated cytosine, that is, cytosine having a methyl group at the position 5.

The "nucleobase sequence" refers to a sequence from the 5' side to the 3' side of nucleobases of each nucleoside contained in an oligonucleotide.

"Consecutive nucleobases" refer to a sequence from the 5' side to the 3' side of nucleobases in a consecutive part of the nucleobase sequence.

The "internucleoside bond" refers to a group or a bond forming a covalent bond between adjacent nucleosides in an oligonucleotide. The "internucleoside bond" include a phosphodiester bond and a "modified internucleoside bond" described below.

The "modified internucleoside bond" refers to a modified phosphodiester bond, and examples thereof include a phosphorothioate bond, a methylphosphonate bond (including a chiral-methylphosphonate bond), a methylthiophosphonate bond, a phosphorodithioate bond, a phosphoramidate bond, a phosphorodiamidate bond, a phosphoramidothioate bond, a boranophosphate bond, and the like. In addition, Journal of Medicinal Chemistry, 2016, 59, pp 9645-9667, Medicinal Chemistry Communication, 2014, 5, pp 1454-1471, Future Medicinal Chemistry, 2011, 3, pp 339-365, and the like disclose examples of the modification of a phosphodiester bond, which can be used as the modified phosphodiester bond.

The "modified nucleoside" refers to a nucleoside having a modified sugar moiety and/or a modified nucleobase.

The "oligonucleotide" refers to a molecule having a structure in which two or more identical or different "nucleosides" are independently linked to each other by the "internucleoside bond" (for example, a phosphodiester bond or a modified phosphodiester bond).

A "modified oligonucleotide" refers to an oligonucleotide containing at least one selected from the group consisting of a modified internucleoside bond, a modified sugar, and a modified nucleobase.

A "compound containing a modified oligonucleotide" refers to a compound containing a modified oligonucleotide in the chemical structure thereof, and the compound may be the modified oligonucleotide per se. Examples of the compound containing a modified oligonucleotide include a compound in which a functional molecule is directly or indirectly bonded to the modified oligonucleotide as described later, a compound containing a prodrug moiety described later, and the modified oligonucleotide per se.

A "DNA" refers to a polynucleotide or an oligonucleotide in which two or more identical or different "deoxyribonucleosides" described above are linked to each other by the "internucleoside bond".

An "RNA" refers to a polynucleotide or an oligonucleotide in which two or more identical or different "ribonucleosides" described above are linked to each other by the "internucleoside bond".

An "antisense effect" means that a target RNA selected corresponding to a target gene hybridizes with, for example, an oligonucleotide having a sequence complementary to a partial sequence of the target RNA to control a function of the target RNA. For example, when the target RNA is mRNA, the antisense effect means inhibition of translation of the target RNA by hybridization, a splicing function converting effect such as exon skipping, degradation of the target RNA by recognition of a portion that has hybridized, and the like.

In one embodiment, the target RNA is a "CALM2 mRNA" and/or a "CALM2 pre-mRNA".

An "antisense oligonucleotide" (ASO) is an oligonucleotide having the antisense effect. Examples thereof include DNA, a gapmer, a mixmer, and the like, but are not limited thereto, and may be RNA, an oligonucleotide designed to usually have the antisense effect, or the like.

The "hybridize" refers to an action of forming a double strand between oligonucleotides including sequences complementary to each other or portions thereof, and a phenomenon that oligonucleotides including sequences complementary to each other or portions thereof form a double strand therebetween.

"Complementary" means that two nucleobases can form a Watson-Crick base pair (natural base pair) or a non-Watson-Crick base pair (Hoogsteen base pair, and the like) via a hydrogen bond. Two oligonucleotides or portions thereof can "hybridize" with each other when their sequences are complementary to each other. In order for two oligonucleotides or portions thereof to hybridize with each other, they need not to be completely complementary to each other, but complementarity for two oligonucleotides or portions thereof to hybridize with each other is preferably 70% or more, more preferably 80% or more, and still more preferably 90% or more (for example, 95%, 96%, 97%, 98%, or 99% or more). Complementarity between sequences is determined by using a computer program that automatically identifies a partial sequence of an oligonucleotide. For example, OligoAnalyzer is one type of such software and is provided by Integrated DNA Technologies. This program can also be used in a website.

The "gapmer" refers to an oligonucleotide containing a "gap segment", a "5' wing segment", and a "3' wing segment" described below.

The "gap segment" is a region containing "at least four consecutive nucleosides recognized by an RNase H", and although the "gap segment" is not particularly limited as long as it contains four or more consecutive nucleosides and is recognized by the RNase H, the consecutive nucleosides are preferably independently selected from a deoxyribonucleoside and a sugar-modified nucleoside. It is preferable that the gap segment contains at least two deoxyribonucleosides, and the nucleosides at the 5' terminal and the 3' terminal of the gap segment are deoxyribonucleosides.

The "5' wing segment" is a region that is linked to the 5' side of the gap segment and does not contain "at least four consecutive nucleosides recognized by the RNase H" described above, but contains "at least one nucleoside". Here, the sugar moiety of the 3' terminal nucleoside of the 5' wing segment is different from the sugar moiety of the 5' terminal nucleoside of the gap segment. The boundary between the 5' wing segment and the gap segment is identified by the difference between the sugar moieties. (For example, the 5' terminal nucleoside of the gap segment is a deoxyribonucleoside, and the 3' terminal nucleoside of the 5' wing segment is a sugar-modified nucleoside.) In general, the 3' terminal nucleoside of the 5' wing segment is a sugar-modified nucleoside. Although the 5' wing segment is not particularly limited as long as the above definition is met, it is preferable that at least one nucleoside described above is independently selected from a deoxyribonucleoside and a sugar-modified nucleoside, and the segment contains at least one sugar-modified nucleoside. It is preferable that the 3' terminal nucleoside of the 5' wing segment is a sugar-modified nucleoside and is linked to the 5' terminal of the gap segment.

The "3' wing segment" is a region that is linked to the 3' side of the gap segment and does not contain "at least four consecutive nucleosides recognized by the RNase H" described above, but contains "at least one nucleoside". Here, the sugar moiety of the 5' terminal nucleoside of the 3' wing segment is different from the sugar moiety of the 3' terminal nucleoside of the gap segment. The boundary between the 3' wing segment and the gap segment is identified by the difference between the sugar moieties. (For example, the 3' terminal nucleoside of the gap segment is a deoxyribonucleoside, and the 5' terminal nucleoside of the 3' wing segment is a sugar-modified nucleoside.) In general, the 5' terminal nucleoside of the 3' wing segment is a sugar-modified nucleoside. Although the 3' wing segment is not particularly limited as long as the above definition is met, it is preferable that at least one nucleoside described above is independently selected from a deoxyribonucleoside and a sugar-modified nucleoside, and the segment contains at least one sugar-modified nucleoside. It is preferable that the 5' terminal nucleoside of the 3' wing segment is a sugar-modified nucleoside and is linked to the 3' terminal of the gap segment.

The "RNase H" is generally known as a ribonuclease that recognizes a double strand in which DNA and RNA in a living body hybridize with each other, and cleaves the RNA to generate single-stranded DNA. RNase H can recognize not only a double strand in which DNA and RNA hybridize with each other but also a double strand in which at least one of a nucleobase moiety, a phosphodiester bond moiety, and a sugar moiety of at least one of DNA and RNA is modified. For example, RNase H can also recognize a double strand in which DNA modified with a phosphorothioate bond and RNA hybridize with each other.

Thus, DNA can be recognized by the RNase H when DNA hybridizes with RNA. In addition, RNA can be cleaved by RNase H when RNA hybridizes with DNA. The same applies to a case where at least one of a nucleobase moiety, a phosphodiester bond moiety, and a sugar moiety is modified in at least one of DNA and RNA. For example, a typical example is an oligonucleotide in which a phosphodiester bond moiety of DNA is modified with phosphorothioate, and the like.

Examples of modification of DNA and/or RNA that can be recognized by the RNase H are described, for example, in Nucleic Acids Research, 2014, 42, pp 5378-5389, Bioorganic & Medicinal Chemistry Letters, 2008, 18, pp 2296-2300, Molecular BioSystems, 2009, 5, pp 838-843, Nucleic Acid Therapeutics, 2015, 25, pp 266-274, The Journal of Biological Chemistry, 2004, 279, pp 36317-36326, and the like.

RNase H used in the present invention is preferably mammalian RNase H, more preferably human RNase H, and particularly preferably human RNase HI.

"At least four consecutive nucleosides recognized by the RNase H" are not particularly limited as long as they include four or more consecutive nucleosides and are recognized by the RNase H, but examples thereof include "at least four consecutive deoxyribonucleosides", and the like. The number of nucleosides constituting "at least four consecutive nucleosides recognized by the RNase H" is, for example, 5 to 30, preferably 5 to 15, more preferably 8 to 12, and particularly preferably 10.

Those skilled in the art can determine whether or not certain at least four certain consecutive nucleosides are "at least four consecutive nucleosides recognized by the RNase H" by the structures of the sugar moieties in the consecutive nucleosides.

"Calmodulin" is a ubiquitously expressed calcium-sensing protein and regulates various proteins including a plurality of ion channels. Calmodulin is associated with various processes such as inflammation, metabolism, apoptosis, muscle contraction, intracellular migration, short-term memory, long-term memory, nerve growth, and an immune response. In particular, calmodulin promotes inactivation of an L-type calcium channel (LTCC) expressed in a heart.

Three different genes, CALM1, CALM2, and CALM3, are known as the genes encoding calmodulin. The three genes encode calmodulin having the same amino acid sequence.

The "CALM2 mRNA" refers to, among the mRNAs encoding calmodulin, the mRNA transcribed from the CALM2 gene and spliced, and the "CALM2 pre-mRNA" refers to, among the pre-mRNAs encoding calmodulin, the pre-mRNA transcribed from the CALM2 gene. The "CALM2 nucleic acid" includes, for example, the "CALM2 mRNA" and the "CALM2 pre-mRNA".

The "CALM2 mRNA" is set forth in, for example, SEQ ID NO: 1, and the "CALM2 pre-mRNA" is set forth in, for example, SEQ ID NO: 2. Both the "CALM2 mRNA" and the "CALM2 pre-mRNA" have nucleosides that are linked to each other by phosphodiester bonds, and thymine is generally substituted by uracil. The "CALM2 mRNA" and the "CALM2 pre-mRNA" do not have any additional modified sugar, modified nucleobase, or modified internucleoside bond.

The "CALM1 mRNA" refers to, among the mRNAs encoding calmodulin, the mRNA transcribed from the CALM1 gene and spliced, and the "CALM1 pre-mRNA" refers to, among the pre-mRNAs encoding calmodulin, the pre-mRNA transcribed from the CALM1 gene. The "CALM1 nucleic acid" includes, for example, the "CALM1 mRNA" and the "CALM1 pre-mRNA".

The "CALM3 mRNA" refers to, among the mRNAs encoding calmodulin, the mRNA transcribed from the CALM3 gene and spliced, and the "CALM3 pre-mRNA" refers to, among the pre-mRNAs encoding calmodulin, the pre-mRNA transcribed from the CALM3 gene. The "CALM3 nucleic acid" includes, for example, the "CALM3 mRNA" and the "CALM3 pre-mRNA".

"Expression of a gene" refers to conversion of coding information in a gene to a structure in a cell or a function thereof. Such structure is not limited, and examples thereof include transcription and translation products (an mRNA, a pre-mRNA, a protein, and the like).

"Long-QT syndrome" (LQTS) is an arrhythmia disease in which a QT interval on the electrocardiogram is prolonged compared to that in a healthy individual. LQTS causes fainting or sudden death by ventricular arrhythmia. The QT interval is the time from the beginning of the QRS wave to the end of the T wave in the electrocardiogram and represents ventricular contraction and relaxation time. Since a normal value of the QT interval varies depending on the heart rate, in general, a corrected QT interval (QTc = QT/√RR) obtained by correcting the QT interval according to the heart rate using Bazett's formula is used. A normal value of the corrected QT interval is from 360 milliseconds to 440 milliseconds. QT prolongation is extension of the corrected QT interval beyond the normal value.

"Congenital long QT syndrome" (congenital LQTS) is a congenital arrhythmia disease among the LQTS and is mainly caused by impairment of an ion channel function. Although most congenital LQTS cases are hereditary (familial), many CALM2 mutations are "de novo mutations", genetic mutations that are not found in the parents. LQT1 to LQT16 have been reported as congenital LQTS, according to the differences in mutated genes. Congenital LQTS is diagnosed by a physician according to a guideline or the like. In general, congenital LQTS is understood as a disease in which a corrected QT interval is prolonged compared to that in a healthy individual, and a congenital LQTS-associated gene has a mutation.

"LQT15" is congenital long QT syndrome caused by a mutation in CALM2. As the CALM2 mutation, a mutation in which the 98^{th} amino acid from the N-terminal of CALM2 is changed from asparagine (N) to serine (S), a mutation in which the 130^{th} amino acid is changed from aspartic acid (D) to glycine (G), and the like are known.

"LQT14" is congenital long QT syndrome caused by a mutation in CALM1, and "LQT16" is congenital long QT syndrome caused by a mutation in CALM3.

"Calmodulinopathy" is a disease caused by a mutation in a gene encoding calmodulin and mainly involves a symptom of arrhythmia. Reported symptoms are long QT syndrome (LQTS), as well as catecholaminergic polymorphic ventricular tachycardia (CPVT), idiopathic ventricular fibrillation (IVF), and the like.

Next, preferred embodiments of the modified oligonucleotide or the compound containing a modified oligonucleotide will be described.

Note that, hereinafter, description will be made using terms with the "modified oligonucleotide", however, it is needless to say that such description is also applicable by suitably replacing the "modified oligonucleotide" with the "compound containing a modified oligonucleotide" or the "antisense oligonucleotide" (ASO), which is the preferred embodiment.

The modified oligonucleotide in the present application preferably targets the CALM2 mRNA and/or pre-mRNA. It is not necessary that the modified oligonucleotide hybridizes with the entire CALM2 mRNA and/or pre-mRNA. In general, as long as the modified oligonucleotide hybridizes with at least a part of the CALM2 mRNA and/or pre-mRNA, a part of the modified oligonucleotide may not be hybridized therewith. For example, the CALM2 gene expression is controlled by hybridization of an oligonucleotide (a gapmer, an oligonucleotide designed to exhibit the normal antisense effect, or the like) having a sequence complementary to a partial sequence of the CALM2 mRNA and/or pre-mRNA with a part of the CALM2 mRNA and/or pre-mRNA.

Complementarity between a nucleobase sequence of the modified oligonucleotide in the present application and the partial sequence of the CALM2 mRNA and/or pre-mRNA is preferably 70% or higher, more preferably 80% or higher, even more preferably 85% or higher, and still even more preferably 90% or higher (for example, 95%, 96%, 97%, 98%, or 99% or higher). Although it is not necessary that the sequence of the modified oligonucleotide in the present application and the partial sequence of the CALM2 mRNA and/or pre-mRNA are completely complementary to each other for the hybridization between the modified oligonucleotide in the present application and at least a part of the partial sequence of the CALM2 mRNA and/or pre-mRNA, it is still even more preferable that the sequences are completely complementary to each other.

The modified oligonucleotide in the present application has a gap segment, a 5' wing segment, and a 3' wing segment.

The gap segment consists of at least five nucleosides independently selected from the group consisting of a deoxyribonucleoside, a ribonucleoside, and a sugar-modified nucleoside, and the 3' terminal and the 5' terminal thereof are each independently deoxyribonucleosides. The gap segment preferably contains "at least four consecutive deoxyribonucleosides".

The number of nucleosides contained in the gap segment is preferably 5 to 30, more preferably 5 to 15, even more preferably 8 to 12, still even more preferably 9 to 11, and particularly preferably 10. In an embodiment, the nucleosides contained in the gap segment are deoxyribonucleosides.

The internucleoside bond contained in the gap segment is preferably independently selected from a phosphodiester bond and a phosphorothioate bond.

It is preferable that at least one of the internucleoside bonds contained in the gap segment is a phosphorothioate bond, more preferable that 50% or more of the bonds are phosphorothioate bonds, even more preferable that 75% or more of the bonds are phosphorothioate bonds, still even more preferable that 80% or more of the bonds are phosphorothioate bonds, still even more preferable that 90% or more of the bonds are phosphorothioate bonds, and particularly preferable that all of the bonds are phosphorothioate bonds.

The 5' wing segment consists of at least one nucleoside independently selected from the group consisting of a deoxyribonucleoside, a ribonucleoside, and a sugar-modified nucleoside, provided that the 3' terminal thereof which is bonded to the gap segment is a sugar-modified nucleoside, and the 5' wing segment does not contain "at least four consecutive nucleosides recognized by the RNase H".

The 3' wing segment consists of at least one nucleoside independently selected from the group consisting of a deoxyribonucleoside, a ribonucleoside, and a sugar-modified nucleoside, provided that the 5' terminal thereof which is bonded to the gap segment is a sugar-modified nucleoside, and the 3' wing segment does not contain "at least four consecutive nucleosides recognized by the RNase H".

It is preferable that the 5' wing segment and the 3' wing segment do not contain "at least four consecutive deoxyribonucleosides".

Hereinafter, common properties of the 5' wing segment and the 3' wing segment will be referred to as the properties of the wing segments.

The numbers of the nucleosides contained in the wing segments are each 1 to 15, preferably 1 to 10, more preferably 1 to 7, even more preferably 2 to 6, still even more preferably 3 to 5, and particularly preferably 3.

The sugar-modified nucleosides contained in the wing segments are preferably nucleosides of which affinity to the partial sequence of the CALM2 mRNA and/or the CALM2 pre-mRNA is increased by substitution or the like or nucleosides of which resistance to a nuclease is increased by substitution or the like. The sugar-modified nucleosides contained in the wing segments are more preferably independently selected from a 2'-modified nucleoside and a 2',4'-BNA.

The 2'-modified nucleoside contained in the wing segments is preferably at least one independently selected from the group consisting of a 2'-O-Me nucleoside, a 2'-O-MOE nucleoside, a 2'-O-AP nucleoside, a 2'-F nucleoside, a 2'-O-NMA nucleoside, a 2'-O-MCE nucleoside, a 2'-DMAECE nucleoside, a 2'-MorECE nucleoside, a 2'-PyECE nucleoside, and a BimECE nucleoside, more preferably at least one independently selected from the group consisting of a 2'-O-Me nucleoside, a 2'-O-MOE nucleoside, a 2'-O-NMA nucleoside, and a 2'-O-MCE nucleoside, even more preferably at least one independently selected from a 2'-O-MOE nucleoside and a 2'-O-MCE nucleoside, and particularly preferably a 2'-O-MCE nucleoside.

The 2',4'-BNA contained in the wing segments is preferably at least one independently selected from the group consisting of an LNA, an ENA, a cEt, a BNA^{NC}, an AmNA, an scpBNA, and a GuNA and more preferably an LNA.

The sugar-modified nucleosides contained in the wing segments are more preferably at least one independently selected from a 2'-O-MOE nucleoside, an LNA, and a 2'-O-MCE nucleoside, even more preferably 2'-O-MOE nucleosides, 2'-O-MCE nucleosides, or an LNA and a 2'-O-MCE nucleoside, and particularly preferably an LNA and a 2'-O-MCE nucleoside.

It is preferable that each of the wing segments consists of 1 to 10 nucleosides independently selected from the group consisting of a sugar-modified nucleoside and a deoxyribonucleoside and contains at least one sugar-modified nucleoside. It is more preferable that each of the wing segments consists of 2 to 6 nucleosides independently selected from the group consisting of a sugar-modified nucleoside and a deoxyribonucleoside and contains at least two sugar-modified nucleosides. It is even more preferable that each of the wing segments consists of 2 to 6 nucleosides independently selected from the group consisting of a 2'-modified nucleoside and a 2',4'-BNA, and still more preferable that each of the wing segments consists of 3 to 5 nucleosides independently selected from the group consisting of a 2'-modified nucleoside and a 2',4'-BNA.

More specifically, each of the wing segments consists of 1 to 10, preferably, 2 to 6 nucleosides independently selected from an LNA, a 2'-O-MCE nucleoside, and a 2'-O-MOE nucleoside, and it is more preferable that each of the wing segments consists of 2 to 5, more preferably, 3 nucleosides independently selected from an LNA and a 2'-O-MCE nucleoside. It is even more preferable that each of the wing segments consists of two LNAs and one or two 2'-O-MCE nucleosides, and particularly preferable that each of the wing segments consists of two LNAs and one 2'-O-MCE nucleoside. As another aspect, it is particularly preferable that each of the wing segments consists of 2 to 5, most preferably 3 LNAs. As still another aspect, it is particularly preferable that each of the wing segments consists of 3 to 6, most preferably 5 2'-O-MCE nucleosides.

As another preferred aspect, each of the wing segments is an oligonucleotide consisting of 2 to 5 nucleosides independently selected from the group consisting of a 2',4'-BNA and a deoxyribonucleoside and containing at least two 2',4'-BNAs. Regarding such oligonucleotide, WO 2016/127002 A or the like can be referred to.

The LNA contained in each of the wing segments is preferably a β-D-methyleneoxy BNA.

In some embodiments, the 5' wing segment and the 3' wing segment each independently contain 3, 4, 5, or 6 sugar-modified nucleosides, and the gap segment consists of 8, 9, 10, 11, 12, 13, or 14 deoxyribonucleosides. The numbers of nucleosides in such gapmer can be represented by ((5' wing segment)-(gap segment)-(3' wing segment)), and includes 5-10-5, 5-11-4, 4-11-5, 4-12-4, 3-14-3, 6-8-6, 3-12-3, 3-10-3, 4-10-4, 3-10-4, 4-10-3, 3-9-3, 4-9-4, 3-9-4, 4-9-3, 3-8-3, 3-8-4, 4-8-3, and 4-8-4.

In some embodiments, the modified oligonucleotide has at least 11, 12, 13, 14, or 15 consecutive nucleosides, the gap segment contains at least 5, 6, 7, 8, or 9 consecutive nucleosides, and the 5' wing segment and the 3' wing segment are any of the following (i) to (iv).
(i) The 5' wing segment and the 3' wing segment each independently contain 3, 4, 5, or 6 2'-O-MOE nucleosides.
(ii) The 5' wing segment and the 3' wing segment each independently contain 3, 4, 5, or 6 2'-O-MCE nucleosides.
(iii) The 5' wing segment and the 3' wing segment each independently contain 2, 3, 4, 5, or 6 LNAs.
(iv) The 5' wing segment and the 3' wing segment each independently contain three or four selected from a 2'-O-MCE nucleoside and an LNA and contain at least one 2'-O-MCE nucleoside and at least one LNA.

The 5' wing segment and the 3' wing segment in the modified oligonucleotide of the present invention are preferably selected from the group consisting of LL, LLL, VLL, LVL, LLV, LVV, VLV, VVL, VVLL, VLVL, LVLV, LLVV, VLLL, LVLL, LLVL, LLLV, LVVV, VLVV, VVLV, and VVVL.

It is more preferable that the 5' wing segment is selected from the group consisting of LL, LLL, VLL, VLV, VVL, VVLL, VLVL, VLLL, VLVV, VVLV, and VVVL, and the 3' wing segment is selected from the group consisting of LL, LLL, LLV, VLV, LVV, LLVV, LVLV, XLLV, VVLV, VLVV, and LVVV.

It is even more preferable that the 5' wing segment is selected from the group consisting of LL, LLL, VLL, VLV, VVL, and VLVL, and the 3' wing segment is selected from the group consisting of LL, LLL, LLV, VLV, LVV, and LVLV.

As another aspect, it is preferable that the 5' wing segment is selected from the group consisting of LLL, VLL, VVL, LVL, and VLVL, and the 3' wing segment is selected from the group consisting of LLL, LLV, LVV, LVL, and LVLV.

It is particularly preferable that the 5' wing segment is LLL, and the 3' wing segment is LLL.

As still another aspect, it is particularly preferable that the 5' wing segment is VLL, and the 3' wing segment is LLV.

Here, L and V in the 5' wing segment and the 3' wing segment are sugar-modified nucleosides having modified sugars that are different from each other, and the left side represents the 5' side, and the right side represents the 3' side. It is preferable that L represents a 2'-4'-bridged nucleoside, and V represents a 2'-modified nucleoside. It is particularly preferable that L represents an LNA, and V represents an MCE nucleoside.

The internucleoside bond contained in the wing segments is preferably independently selected from a phosphodiester bond and a phosphorothioate bond.

It is preferable that at least one of the internucleoside bonds contained in the 5' wing segment is a phosphorothioate bond, preferable that 50% or more of the bonds are phosphorothioate bonds, more preferable that 60% or more of the bonds are phosphorothioate bonds, even more preferable that 75% or more of the bonds are phosphorothioate bonds, still even more preferable that 80% or more of the bonds are phosphorothioate bonds, still even more preferable that 90% or more of the bonds are phosphorothioate bonds, and particularly preferable that all of the bonds are phosphorothioate bonds.
Description of the bonds in the 3' wing segment is the same as that of the 5' wing segment.

As another aspect, it is preferable that all of the internucleoside bonds contained in the wing segments are phosphodiester bonds, from the viewpoint that toxicity can be reduced.

In the present application, it is preferable that the 3' terminal of the 5' wing segment and the 5' terminal of the gap segment in the modified oligonucleotide are linked to each other by forming a phosphodiester bond or a modified phosphodiester bond, and the 5' terminal of the 3' wing segment and the 3' terminal of the gap segment are linked to each other by forming a phosphodiester bond or a modified phosphodiester bond. It is more preferable that the 3' terminal of the 5' wing segment and the 5' terminal of the gap segment are linked to each other by forming a modified phosphodiester bond, and the 5' terminal of the 3' wing segment and the 3' terminal of the gap segment are linked to each other by forming a modified phosphodiester bond. It is even more preferable that the 3' terminal of the 5' wing segment and the 5' terminal of the gap segment are linked to each other by a phosphorothioate bond, and the 5' terminal of the 3' wing segment and the 3' terminal of the gap segment are linked to each other by forming a phosphorothioate bond.

The modified oligonucleotide in the present application is a modified oligonucleotide with a length of 8 to 80, preferably 11 to 50, more preferably 15 to 25, even more preferably 16 to 20, and particularly preferably 16 consecutive nucleosides.

The modified oligonucleotide in the present application preferably has a nucleobase sequence containing at least 8, more preferably, 8, 9, 10, 11, 12, 13, 14, or 15 consecutive nucleobases contained in a nucleobase sequence of any one of SEQ ID NOs: 3 to 73.

The modified oligonucleotide in the present application more preferably has a nucleobase sequence containing a nucleobase sequence of any one of SEQ ID NOs: 3 to 73.

The modified oligonucleotide in the present application even more preferably has a nucleobase sequence consisting of a nucleobase sequence of any one of SEQ ID NOs: 3 to 73.

In one embodiment, the modified oligonucleotide in the present application preferably has a nucleobase sequence containing at least 8, more preferably, 8, 9, 10, 11, 12, 13, 14, or 15 consecutive nucleobases contained in a nucleobase sequence of any one of SEQ ID NOs: 3 to 30, 32 to 36, 38 to 46, and 48 to 71.

In one embodiment, the modified oligonucleotide in the present application more preferably has a nucleobase sequence containing a nucleobase sequence of any one of SEQ ID NOs: 3 to 30, 32 to 36, 38 to 46, and 48 to 71.

In one embodiment, the modified oligonucleotide in the present application even more preferably has a nucleobase sequence consisting of a nucleobase sequence of any one of SEQ ID NOs: 3 to 30, 32 to 36, 38 to 46, and 48 to 71.

In another embodiment, the modified oligonucleotide in the present application preferably has a nucleobase sequence containing at least 8, more preferably, 8, 9, 10, 11, 12, 13, 14, or 15 consecutive nucleobases contained in a nucleobase sequence of any one of SEQ ID NOs: 3 to 20.

In another embodiment, the modified oligonucleotide in the present application more preferably has a nucleobase sequence containing a nucleobase sequence of any one of SEQ ID NOs: 3 to 20.

In another embodiment, the modified oligonucleotide in the present application even more preferably has a nucleobase sequence consisting of a nucleobase sequence of any one of SEQ ID NOs: 3 to 20.

In still another embodiment, the nucleobase sequence of the modified oligonucleotide in the present application preferably contains at least 8, more preferably, 8, 9, 10, 11, 12, 13, 14, or 15 consecutive nucleobases contained in the nucleobase sequence of SEQ ID NO: 4.

In still another embodiment, the nucleobase sequence of the modified oligonucleotide in the present application preferably contains at least the nucleobase sequence of SEQ ID NO: 4.

In still another embodiment, the modified oligonucleotide in the present application even more preferably has a nucleobase sequence consisting of the nucleobase sequence of SEQ ID NO: 4.

It is preferable that the modified oligonucleotide in the present application is complementary to at least a part of a nucleobase sequence selected from the group consisting of the nucleobase sequences indicated by nucleobase position nos. 102 to 117, 160 to 175, 183 to 205, 212 to 227, 322 to 337, 365 to 405, 411 to 434, 464 to 494, 506 to 521, 606 to 635, 636 to 651, 692 to 707, 715 to 749, 754 to 801, 829 to 857, 862 to 934, 951 to 970, 995 to 1010, 1006 to 1021, 1036 to 1051, 1062 to 1077, 1081 to 1104, 1138 to 1166, 1188 to 1203, and 1239 to 1254 in the CALM2 mRNA set forth in SEQ ID NO: 1.

In one embodiment, it is preferable that the modified oligonucleotide in the present application is complementary to at least a part of a nucleobase sequence selected from the group consisting of the nucleobase sequences indicated by nucleobase position nos. 102 to 117, 160 to 175, 183 to 198, 212 to 227, 322 to 337, 365 to 380, 387 to 402, 479 to 494, 620 to 635, 715 to 730, 862 to 877, 896 to 934, 995 to 1010, 1062 to 1077, 1089 to 1104, 1140 to 1166, and 1239 to 1254 in the CALM2 mRNA set forth in SEQ ID NO: 1.

It is more preferable that the modified oligonucleotide in the present application is complementary to at least a part of the nucleobase sequence indicated by nucleobase position nos. 896 to 934 in the CALM2 mRNA set forth in SEQ ID NO: 1.

It is still more preferable that the modified oligonucleotide in the present application is complementary to at least a part of the nucleobase sequence indicated by nucleobase position nos. 907 to 922 in the CALM2 mRNA set forth in SEQ ID NO: 1.

Complementarity between the modified oligonucleotide in the present application and the portion of the CALM2 mRNA set forth in SEQ ID NO: 1 (for example, each of the target regions described above) is at least 80% or higher, preferably 85% or higher, more preferably 90% or higher, and even more preferably 95%, 96%, 97%, 98%, or 99% or higher.

It is preferable that the modified oligonucleotide in the present application is complementary to at least a part of a nucleobase sequence selected from the group consisting of the nucleobase sequences indicated by nucleobase position nos. 102 to 117, 160 to 175, 1624 to 1639, 1661 to 1676, 3355 to 3370, 5859 to 5881, 13959 to 13974, 14069 to 14084, 14228 to 14278, 14284 to 14307, 14764 to 14794, 14806 to 14821, 15824 to 15869, 15910 to 15925, 15933 to 15967, 15972 to 16019, 16047 to 16075, 16080 to 16152, 16169 to 16188, 16213 to 16239, 16254 to 16269, 16280 to 16295, 16299 to 16322, 16356 to 16384, 16406 to 16421, and 16457 to 16472 in the CALM2 pre-mRNA set forth in SEQ ID NO: 2.

In one embodiment, it is preferable that the modified oligonucleotide in the present application is complementary to at least a part of a nucleobase sequence selected from the group consisting of the nucleobase sequences indicated by nucleobase position nos. 102 to 117, 160 to 175, 5859 to 5874, 13959 to 13974, 14069 to 14084, 14238 to 14253, 14260 to 14275, 14779 to 14794, 15838 to 15853, 15933 to 15948, 16080 to 16095, 16114 to 16152, 16213 to 16228, 16280 to 16295, 16307 to 16322, 16358 to 16384, and 16457 to 16472 in the CALM2 pre-mRNA set forth in SEQ ID NO: 2.

It is more preferable that the modified oligonucleotide in the present application is complementary to at least a part of a nucleobase sequence selected from the group consisting of the nucleobase sequence indicated by nucleobase position nos. 16114 to 16152 in the CALM2 pre-mRNA set forth in SEQ ID NO: 2.

It is more preferable that the modified oligonucleotide in the present application is complementary to at least a part of the nucleobase sequence indicated by nucleobase position nos. 16125 to 16140.

Complementarity between the modified oligonucleotide in the present application and the portion of the CALM2 pre-mRNA set forth in SEQ ID NO: 2 (for example, each of the target regions described above) is at least 80% or higher, preferably 85% or higher, more preferably 90% or higher, and even more preferably 95%, 96%, 97%, 98%, or 99% or higher.

The modified oligonucleotide with a length of 8 to 80 consecutive nucleosides having a nucleobase sequence containing at least 8 consecutive nucleobases contained in a nucleobase sequence of any one of SEQ ID NOs: 3 to 73 (SEQ ID NOs: 3 to 20 in one embodiment) preferably has 2 to 5 nucleosides selected from the group consisting of an LNA and a 2'-O-MCE nucleoside, more preferably has one or two LNAs and one or two 2'-O-MCE nucleosides, even more preferably has two LNAs and one 2'-O-MCE nucleoside, and particularly preferably has three LNAs in each of the wing segments.

A functional molecule may be directly or indirectly bonded to the modified oligonucleotide in the present application. The functional molecule and the modified oligonucleotide may be directly bonded to each other or indirectly bonded to each other through another substance, and it is preferable that the oligonucleotide and the functional molecule are bonded to each other by a covalent bond, an ionic bond, or a hydrogen bond. It is more preferable that the oligonucleotide and the functional molecule are directly bonded to each other by a covalent bond or bonded to each other by a covalent bond through a linker (linking group), from the viewpoint of high stability of the bond.

In a case where the functional molecule is bonded to the modified oligonucleotide by a covalent bond, the functional molecule is preferably directly or indirectly bonded to the 3' terminal or 5' terminal of a modified oligonucleotide molecule. The bond between the linker or functional molecule and the terminal nucleoside of the modified oligonucleotide molecule is selected depending on the functional molecule.

The linker or functional molecule and the terminal nucleoside of the modified oligonucleotide molecule are preferably linked by a phosphodiester bond or a modified phosphodiester bond and more preferably linked by a phosphodiester bond.

The linker or functional molecule may be directly linked to the oxygen atom at the position 3' in the 3' terminal nucleoside of the modified oligonucleotide molecule or the oxygen atom at the position 5' in the 5' terminal nucleoside of the modified oligonucleotide molecule.

A structure of the "functional molecule" (conjugate) is not particularly limited, and a desired function is imparted to the modified oligonucleotide by the bonding of the functional molecule thereto. Examples of the desired function include a labeling function, a purifying function, and a delivery function to a target site. Examples of a molecule imparting the labeling function include compounds such as a fluorescent protein and luciferase. Examples of a molecule imparting the purifying function include compounds such as biotin, avidin, the His-tag peptide, the GST-tag peptide, the FLAG tag peptide, and the like.

Furthermore, from the viewpoints of efficiently delivering the modified oligonucleotide of the present application to a target site (for example, heart) with high specificity and very effectively controlling the CALM2 gene expression by the modified oligonucleotide, it is preferable that, as the functional molecule, a molecule having the function of delivering the modified oligonucleotide to the target site is bonded to the modified oligonucleotide. Regarding the molecule having the delivery function, for example, European Journal of Pharmaceutics and Biopharmaceutics, 2016, 107, pp 321-340, Advanced Drug Delivery Reviews, 2016, 104, pp 78-92, Expert Opinion on Drug Delivery, 2014, 11, pp 791-822, Nucleic Acids Research, 2019, 47, 12, pp 6045-6058, WO 2017/053995 A, and the like can be referred to.

Examples of the functional molecule include a sugar, a lipid, a peptide, a protein, derivatives thereof, and the like.

Examples of the functional molecule that can efficiently deliver the modified oligonucleotide of the present application to the heart or the like include a lipid. Examples of the lipid include cholesterol; vitamins such as vitamin E (tocopherols and tocotrienols), vitamin A, vitamin D, and vitamin K; steroids such as a glucocortiroid, a mineralcortiroid, estrogen, androgen, and progesterone; a fatty acid such as a C5-30 saturated fatty acid and a C5-30 unsaturated fatty acid; an intermediate metabolite such as an acylcarnitine and an acyl-CoA; a glycolipid; a glyceride; derivatives thereof, and the like.

The lipid that is used is preferably a fatty acid such as a C5-30 saturated fatty acid, a C5-30 unsaturated fatty acid, and the like. Here, the C5-30 saturated fatty acid is a linear or branched saturated fatty acid having 5 to 30 carbon atoms, and specific examples thereof include palmitic acid, stearic acid and the like. The C5-30 unsaturated fatty acid is a linear or branched unsaturated fatty acid having 5 to 30 carbon atoms and at least one carbon-carbon double bond, and specific examples thereof include oleic acid, linoleic acid, α-linolenic acid, γ-linolenic acid, arachidonic acid, icosapentaenoic acid, docosahexaenoic acid, and the like.

The lipid that is used is more preferably a C15-25 saturated fatty acid or a C15-25 unsaturated fatty acid and even more preferably a C15-25 saturated fatty acid. The C15-25 saturated fatty acid is a linear or branched saturated fatty acid having 15 to 25 carbon atoms, and the C15-25 unsaturated fatty acid is a linear or branched unsaturated fatty acid having 15 to 25 carbon atoms and at least one carbon-carbon double bond. The lipid that is used is even more preferably a linear fatty acid among the C15-25 saturated fatty acids or the C15-25 unsaturated fatty acids, still even more preferably a linear C15-25 saturated fatty acid, and particularly preferably palmitic acid.

Another lipid that is used is preferably cholesterol or vitamin E, more preferably tocopherols, even more preferably a tocopherol, and particularly preferably an α-tocopherol, from the viewpoint of ameliorating accumulation in the heart.

As a functional molecule that can deliver the modified oligonucleotide of the present application to liver with high specificity, a sugar derivative which interacts with an asialoglycoprotein receptor may be used.

The "asialoglycoprotein receptor" is present on the surface of a liver cell and has an activity of recognizing a galactose residue of an asialoglycoprotein and degrading the molecule by cellular uptake. The "sugar derivative which interacts with an asialoglycoprotein receptor" is preferably a compound that has a structure similar to that of a galactose residue and undergoes cellular uptake by the interaction with the asialoglycoprotein receptor, and examples thereof include a GalNAc (N-acetylgalactosamine) derivative, a galactose derivative, and a lactose derivative.

Examples of the functional molecule that can efficiently deliver the modified oligonucleotide of the present application to each organ with high specificity by interaction with various proteins present on surfaces of cells in the organ include a ligand of a receptor, an antibody, and a peptide or protein of their fragments.

The functional molecule and the modified oligonucleotide may be bonded to each other through a linker. Since any linker that enables the modified oligonucleotide molecule to exhibit the function of the functional molecule may be used, there is no particular limitation on the linker as long as it can stably bond the functional molecule to the oligonucleotide. Examples of the linker include a group derived from an oligonucleotide having 1 to 20 nucleosides or a nucleoside, a group derived from a polypeptide having 1 to 20 amino acids or an amino acid, an alkylene having 1 to 20 carbon atoms, an alkenylene having 2 to 20 carbon atoms, and a polyalkylene glycol group. The group derived from an oligonucleotide having 1 to 20 nucleosides or a nucleoside is a divalent group formed by removing a hydrogen atom or the like from each of the 3' terminal and the 5' terminal of the oligonucleotide having 1 to 20 nucleoside or the nucleoside (nucleoside in a case where the number of the nucleosides is 1). Regarding the group derived from an oligonucleotide having 1 to 20 nucleosides or a nucleoside, for example, WO 2017/053995 A can be referred to. WO 2017/053995 A describes, for example, a three-base linker having a TCA motif and a one- to five-base linker not having a TCA motif. The group derived from a polypeptide having 1 to 20 amino acids or an amino acid is a divalent group formed by removing two groups selected from a hydroxy, a hydrogen atom, and an amino from the polypeptide having 1 to 20 amino acids or the amino acid (amino acid in a case where the number of the amino acids is 1). The alkylene and alkenylene may be linear or branched. The polyalkylene glycol group may be, for example, a group formed by removing two selected from a hydroxy group and a hydrogen atom of a hydroxy group from a polyalkylene glycol represented by Formula:

H-(O-W-)n-OH

(In the formula, W is independently an alkylene having 1 to 10 carbon atoms, where the alkylene may be independently substituted by a hydroxy and/or an amino, and n is an integer of 1 to 20).

It is preferable that W is independently an alkylene having 2 to 4 carbon atoms. Here, the alkylene may be independently substituted by a hydroxy and/or an amino. It is more preferable that W is independently an alkylene having 2 or 3 carbon atoms. Here, the alkylene may be independently substituted by a hydroxy. It is even more preferable that W is independently ethane-1,2-diyl or propane-1,2-diyl. Here, the ethane-1,2-diyl and propane-1,2-diyl may be substituted by a hydroxy group. n is preferably an integer of 1 to 10, more preferably an integer of 1 to 6, even more preferably an integer of 2 to 5, and particularly preferably 4.

The linker mediating the bonding between the functional molecule and the modified oligonucleotide is preferably an alkylene having 1 to 20 carbon atoms or an alkenylene having 2 to 20 carbon atoms, more preferably an alkylene having 2 to 10 carbon atoms, even more preferably an alkylene having 6 carbon atoms, and particularly preferably a 1,6-hexane-diyl group. Another preferable linker is a polyalkylene glycol group.

The compound containing the modified oligonucleotide in the present application may contain a prodrug moiety.

A prodrug is a derivative of a drug compound having a group that can be chemically or metabolically degraded and is a compound which is induced to become a pharmacologically active drug compound by solvolysis or degradation in vivo under a physiological condition. Methods for selecting and producing a suitable prodrug derivative are described in, for example, Design of Prodrugs (Elsevier, Amsterdam, 1985). Examples of a prodrug moiety, such as an acyloxy derivative, produced by causing a reaction between a hydroxy group of the modified oligonucleotide (or the compound containing the modified oligonucleotide) and a suitable acyl halide, a suitable acid anhydride, or a suitable halogenated alkyloxycarbonyl compound include - O-C(=O)C₂H₅, -O-C(=O)(t-Bu), -O-C(=O)C₁₅H₃₁, -O-C(=O)-(m-CO₂Na-Ph), -O-C(=O)CH₂CH₂CO₂Na, -OC(=O)CH(NH₂)CH₃, -O-C(=O)CH₂N(CH₃)₂, -O-CH₂OC(=O)CH₃, and the like.

The prodrug of the compound containing the modified oligonucleotide in the present application includes a complex formed by hybridization of two or more oligonucleotides. Examples of such preferable structure (prodrug) include a doublestranded oligonucleotide which includes an oligonucleotide including a ribonucleoside (for example, RNA), an oligonucleotide including a peptide nucleic acid (PNA), or an oligonucleotide including a deoxyribonucleoside (for example, DNA), which are complementary to a modified oligonucleotide (or a compound containing a modified oligonucleotide) (for example, WO 2013/089283 A, WO 2017/068791 A, WO 2017/068790 A, or WO 2018/003739 A). Examples of the preferable structure (prodrug) include a single-stranded oligonucleotide in which an RNA complementary to a modified oligonucleotide is bonded by a linker (for example, WO 2017/131124 A or WO 2018/143475 A), and the like. The linker may be an oligonucleotide linker or a linker including a non-nucleoside structure. Examples of the preferable structure (prodrug) also include a single-stranded oligonucleotide in which an RNA complementary to the modified oligonucleotide is directly linked (WO 2019/022196 A).

The compound containing the modified oligonucleotide in the present application also includes a compound in which two identical or different modified oligonucleotides are linked to each other. Regarding the structure in which two modified oligonucleotides are linked to each other, for example, WO 2017/131124 A and WO 2018/143475 A can be referred to.

The compound containing the modified oligonucleotide in the present application includes those that exist by undergoing tautomerization or geometric isomerization, as well as those that exist as a mixture thereof or a mixture of the respective isomers thereof. Moreover, in a case where an asymmetric center exists or an asymmetric center is generated as a result of isomerization, the compound containing the modified oligonucleotide in the present application also includes those that exist as an optical isomer or a mixture of optical isomers at an arbitrary ratio. Furthermore, in a case of a compound having two or more asymmetric centers, diastereomers also exist due to optical isomerism at each center. The present invention includes a mixture containing all of such forms at an arbitrary ratio. In addition, optically active substances can be obtained by a well-known method for this purpose.

For example, in a case where the compound containing the modified oligonucleotide in the present application contains a modified phosphodiester bond (for example, a phosphorothioate bond), and the phosphorus atom is an asymmetric atom, both forms of an oligonucleotide in which conformation around the phosphorus atoms is controlled and an oligonucleotide in which the conformation around the phosphorus atoms is not controlled are included in the scope of the present invention.

The compound containing the modified oligonucleotide in the present application and a prodrug or pharmaceutically acceptable salt thereof can exist as any crystalline form and any hydrate according to a production condition, and the crystalline forms or hydrates and a mixture thereof are also included in the scope of the present invention. Moreover, the compound containing the modified oligonucleotide in the present application and a prodrug or pharmaceutically acceptable salt thereof may exist as a solvate containing an organic solvent such as acetone, ethanol, 1-propanol, and 2-propanol, and all of such forms are included in the scope of the present invention.

The compound containing the modified oligonucleotide in the present application can be converted to a pharmaceutically acceptable salt or isolated from the generated salt as necessary. Examples of the pharmaceutically acceptable salt of the compound include salts formed with an alkali metal (lithium, sodium, potassium, or the like), an alkaline earth metal (calcium or the like), magnesium, ammonium, an organic base (triethylamine, trimethylamine, or the like), an amino acid (glycine, lysine, glutamic acid, or the like), an inorganic acid (hydrochloric acid, hydrobromic acid, phosphoric acid, sulfuric acid, or the like), or an organic acid (acetic acid, citric acid, maleic acid, fumaric acid, tartaric acid, benzenesulfonic acid, methanesulfonic acid, p-toluenesulfonic acid, or the like).

In particular, a salt may be formed with an alkali metal (lithium, sodium, potassium, or the like), an alkaline earth metal (calcium or the like), magnesium, ammonium, or the like, by conversion of a partial structure represented by -P(=O)(OH)-to an anionic partial structure represented by -P(=O)(O⁻)-. Similarly, a salt may also be formed with an alkali metal, an alkaline earth metal, ammonium, or the like, by conversion of a partial structure represented by -P(=O)(SH)-, which forms a phosphorothioate bond, to an anionic partial structure represented by -P(=O)(S⁻)-. Salts are formed in a similar manner in cases of other modified phosphodiester bonds as well.

The pharmaceutically acceptable salt is particularly preferably a sodium salt.

The compound containing the modified oligonucleotide in the present application can be prepared by suitably selecting a method known to those skilled in the art. For example, those skilled in the art can design a nucleoside sequence of the modified oligonucleotide based on nucleoside sequence information of a target RNA, and synthesize the modified oligonucleotide using a commercially available automated nucleic acid synthesizer (manufactured by Applied Biosystems, Inc., manufactured by Beckman Coulter, Inc., manufactured by GeneDesign, Inc., or the like). The synthesis can also be performed by a reaction using an enzyme. Examples of the enzyme include, but are not limited to, a polymerase, a ligase, and a restriction enzyme. In other words, the method for producing the compound containing the modified oligonucleotide in the present application can include a step of elongating a nucleoside chain at the 3' terminal or the 5' terminal.

A number of methods for bonding an oligonucleotide to a functional molecule are well known in the field, and, for example, European Journal of Pharmaceutics and Biopharmaceutics, 2016, 107, pp 321-340, Advanced Drug Delivery Reviews, 2016, 104, pp 78-92, Expert Opinion on Drug Delivery, 2014, 11, pp 791-822, and the like can be referred to. For example, the functional molecule and the linker can be bonded to each other by a known method, which are then induced to become an amidite form by an amiditation reagent or an H-phosphonate form by an H-phosphonate reagent and bonded to the oligonucleotide.

The compound containing the modified oligonucleotide in the present application can be prepared by purifying the obtained oligonucleotide by reversed-phase column chromatography or the like.

The compound containing the modified oligonucleotide in the present application or a pharmaceutically acceptable salt thereof can be included in a drug as an active ingredient.

Since the compound containing the modified oligonucleotide in the present application or a pharmaceutically acceptable salt thereof can effectively inhibit the CALM2 gene expression, it can be used in a drug for treating, preventing, and/or ameliorating a disease or a condition against which an inhibitory action on the CALM2 gene expression is effective. The disease that can be treated, prevented, and/or ameliorated by the drug which contains the compound containing the modified oligonucleotide in the present application or a pharmaceutically acceptable salt thereof is not particularly limited as long as it is a disease against which the inhibitory action on the CALM2 gene expression is effective, and examples thereof include congenital long QT syndrome (especially LQT15), calmodulinopathy, and other diseases associated with calmodulin such as an inflammatory disease, a metabolic disease, diseases associated with apoptosis, muscle contraction, and intracellular migration of calcium ions or the like, diseases associated with short-term memory and/or long-term memory, a disease associated with nerve growth, and an immune disease. The compound containing the modified oligonucleotide in the present application or a pharmaceutically acceptable salt thereof is particularly effective in treating, preventing, and/or ameliorating congenital long QT syndrome and even calmodulinopathy.

The present application can also provide a composition which contains the compound containing the modified oligonucleotide as an active ingredient and is used to, for example, inhibit the CALM2 gene expression by the antisense effect. The compound containing the modified oligonucleotide in the present application is particularly useful as an ingredient of a pharmaceutical composition for treating, preventing, and/or ameliorating a disease against which the inhibition of the CALM2 gene expression is effective, such as congenital long QT syndrome (especially LQT15) and even calmodulinopathy.

The pharmaceutical composition which contains the compound containing the modified oligonucleotide in the present application or a pharmaceutically acceptable salt thereof can be formulated by a known drug formulation method. For example, the pharmaceutical composition can be enterally (for example, orally, or the like) or nonenterally (parenterally) used as an injection, a capsule, a tablet, a pill, a liquid, a powder, granules, fine granules, a film coating agent, pellets, a lozenge, a sublingual formulation, a chewable formulation, a buccal formulation, a paste, a syrup, a suspending agent, an elixir, an emulsion, a liniment, an ointment, a plaster, a cataplasm, a transdermal formulation, a lotion, an inhalant, an aerosol, a suppository, or the like.

During the formulation, a pharmaceutically acceptable carrier, specifically, sterile water or physiological saline, vegetable oil, a solvent, a base, an emulsifier, a suspending agent, a surfactant, a pH adjuster, a stabilizer, a flavoring agent, an aromatic agent, an excipient, a vehicle, an antiseptic agent, a binder, a diluent, an isotonizing agent, an analgesic agent, an expander, a disintegrant, a buffer, a coating agent, a lubricant, a colorant, a sweetening agent, a thickening agent, a taste and odor masking agent, a solubilizing agent, and other additives can be suitably combined.

A route of administration of the pharmaceutical composition which contains the compound containing the modified oligonucleotide in the present application or a pharmaceutically acceptable salt thereof is not particularly limited, and examples thereof include enteral (for example, oral, or the like) or non-enteral administration. More preferable examples of the route of administration include oral administration, intravenous administration, intraarterial administration, intraperitoneal administration, subcutaneous administration, intradermal administration, intratracheal administration, rectal administration, intramuscular administration, intrathecal administration, intraventricular administration, transnasal administration, intravitreal administration, and administration by infusion, even more preferable examples include intravenous administration and subcutaneous administration, and particularly preferable examples include intravenous administration.

The pharmaceutical composition (preparation) for subcutaneous administration or intravenous administration which contains the compound containing the modified oligonucleotide in the present application or a pharmaceutically acceptable salt thereof can be produced by a conventional method. For example, a preparation obtained by adding an additive such as a buffer and an isotonizing agent to the compound containing the modified oligonucleotide in the present application or a pharmaceutically acceptable salt thereof is completely dissolved in water for injection and then filtered and sterilized. When this preparation solution fills a sterilized syringe, fills a sterilized vial, or is lyophilized after filling a sterilized vial, a prefilled syringe, an injectable preparation, or a preparation to be prepared before use can be produced, respectively. Instead of the filtration and sterilization, terminal sterilization such as autoclaving and gamma-ray radiation may be performed.

Diseases or conditions of various mammals can be treated, prevented, and/or ameliorated by the compound containing the modified oligonucleotide in the present application or a pharmaceutically acceptable salt thereof. For example, diseases or conditions of mammals including a human, a cow, a sheep, a goat, a horse, a dog, a cat, a guinea pig, a rat, a rabbit, and a chimpanzee, or other bovine species, ovine species, equine species, canine species, feline species, and rodent species such as a mouse can be treated, prevented, and/or ameliorated, and the examples of the mammals are not limited thereto. The mammal is particularly preferably a human.

In a case of administering the compound containing the modified oligonucleotide in the present application or a pharmaceutically acceptable salt thereof to a mammal such as a human, a dose of the compound or a pharmaceutically acceptable salt thereof is preferably an effective dose. The effective dose refers to a sufficient amount of a compound for achieving a desired pharmacological effect in an individual who is in need of the drug and varies depending on the age, weight, symptom, and health status of the individual in which the disease or condition needs to be treated, prevented, and/or ameliorated, and the specific type of the compound or a pharmaceutically acceptable salt thereof to be used or the amount of the compound or a pharmaceutically acceptable salt thereof blended in a pharmaceutical composition, and thus, the effective dose is suitably selected. The dose is preferably 0.0001 mg/kg/day to 100 mg/kg/day in terms of the modified oligonucleotide in the present application (particularly, an ASO).

Examples of a preferred embodiment of the use of the modified oligonucleotide in the present application or a pharmaceutically acceptable salt thereof include the following.

A method of controlling the CALM2 gene expression, the method comprising a step of bringing the compound containing the modified oligonucleotide in the present application or a pharmaceutically acceptable salt thereof into contact with cells.

A method of controlling the CALM2 gene expression in a subject, the method comprising a step of administering an effective dose of a pharmaceutical composition which contains the compound containing the modified oligonucleotide in the present application or a pharmaceutically acceptable salt thereof to a subject in need of the pharmaceutical composition.

Use of the compound containing the modified oligonucleotide in the present application or a pharmaceutically acceptable salt thereof for controlling the CALM2 gene expression.

Use of the compound containing the modified oligonucleotide in the present application or a pharmaceutically acceptable salt thereof for production of a drug for controlling the CALM2 gene expression.

The compound containing the modified oligonucleotide in the present application or a pharmaceutically acceptable salt thereof for controlling the CALM2 gene expression.

### [Examples]

Hereinafter, the present invention will be described in greater detail based on Examples, however, the following Examples do not limit the scope of the present invention in any manner, and the scope of the present invention should be determined based on the attached claims.

In Examples, "Compd No" and "Chemical Structure" in the tables (Tables 1 to 12) stand for the compound number and the chemical structure of each compound, respectively.

In the sequences indicated in Examples (Tables 1, 5, 6, and 10), "(L)" represents an LNA (β-D-methyleneoxy BNA), "(V)" represents a 2'-O-MCE nucleoside, a lowercase alphabet represents a deoxyribonucleoside, "^" represents a phosphorothioate bond, "5(x)" indicates that the nucleobase of the deoxyribonucleoside is 5-methylcytosine, and "5" in "5(V)" and "5(L)" indicates that the nucleobase of the nucleoside is 5-methylcytosine, unless otherwise described.

### [Production Example 1]

Antisense oligonucleotides indicated in Table 1 (compounds represented by the chemical structures corresponding to the compound numbers) were prepared using an automated nucleic acid synthesizer nS-8II (manufactured by GeneDesign, Inc.).

**[Table1]**

| Compd No | Chemical Structure |
|---|---|
| P19710001 | G(L)^G(L)^T(L)^5(x)^t^t^5(x)^a^5(x)^t^t^t^g^5(L)^T(L)^G(L) |
| P19710002 | 5(L)^5(L)^5(L)^a^g^a^t^g^t^t^5(x)^5(x)^5(x)^T(L)^5(L)^A(L) |
| P19710003 | A(L)^A(L)^G(L)^5(x)^g^5(x)^t^a^5(x)^5(x)^g^g^t^T(L)^T(L)^5(L) |
| P19710004 | G(L)^T(L)^G(L)^t^a^5(x)^g^a^g^t^a^5(x)^5(x)^T(L)^G(L)^G(L) |
| P19710006 | G(L)^G(L)^A(L)^5(x)^t^a^a^t^t^5(x)^g^5(x)^5(x)^T(L)^5(L)^5(L) |
| P19710007 | G(L)^T(L)^5(L)^5(x)^a^t^a^g^t^5(x)^5(x)^a^5(x)^G(L)^5(L)^A(L) |
| P19710008 | G(L)^5(L)^A(L)^g^a^t^t^t^t^a^a^g^t^G(L)^G(L)^5(L) |
| P19710009 | A(L)^G(L)^T(L)^5(x)^a^g^t^t^g^g^t^5(x)^a^G(L)^5(L)^5(L) |
| P19710010 | G(L)^G(L)^G(L)^a^a^g^t^5(x)^a^a^t^t^g^T(L)^G(L)^5(L) |
| P19710011 | T(L)^T(L)^T(L)^g^a^5(x)^a^g^t^a^g^g^g^A(L)^G(L)^A(L) |
| P19710012 | G(L)^5(L)^A(L)^g^a^g^5(x)^a^a^5(x)^5(x)^a^t^T(L)^G(L)^G(L) |
| P19710014 | T(L)^5(L)^A(L)^t^g^t^5(x)^5(x)^t^g^t^a^a^5(L)^T(L)^5(L) |
| P19710015 | G(L)^5(L)^A(L)^g^a^a^g^g^g^5(x)^t^t^a^G(L)^A(L)^T(L) |
| P19710016 | G(L)^T(L)^G(L)^g^5(x)^t^a^a^a^5(x)^a^a^a^G(L)^T(L)^T(L)^ |
| P19710017 | G(L)^5(L)^T(L)^t^5(x)^t^t^t^g^a^a^t^t^5(L)^T(L)^G(L) |
| P19710018 | T(L)^5(L)^T(L)^g^5(x)^a^g^5(x)^a^5(x)^t^a^a^T(L)^A(L)^T(L) |
| P19710019 | T(L)^G(L)^5(L)^5(x)^a^t^5(x)^a^t^t^g^t^5(x)^A(L)^G(L)^A(L) |
| P19710020 | G(L)^T(L)^A(L)^5(x)^a^g^g^a^5(x)^5(x)^a^g^5(x)^A(L)^G(L)^T(L) |

Positions in the human CALM2 mRNA or pre-mRNA sequence targeted by each of the antisense oligonucleotides indicated in Table 1 and SEQ ID NO and the nucleobase sequence corresponding to each of the antisense oligonucleotides are indicated in Table 2.

Regarding the sequences indicated in Table 2, "SEQ1 START" stands for the "start site in SEQ ID NO: 1", and indicates the position number of the nucleoside on a side closest to a 5' side targeted by an antisense oligonucleotide in the human CALM2 mRNA sequence. "SEQ1 END" stands for the "end site in SEQ ID NO: 1", and indicates the position number of the nucleoside on a side closest to a 3' side targeted by an antisense oligonucleotide in the human CALM2 mRNA sequence.

"SEQ2 START" stands for the "start site in SEQ ID NO: 2", and indicates the position number of the nucleoside on a side closest to a 5' side targeted by an antisense oligonucleotide in the human CALM2 pre-mRNA sequence. "SEQ2 END" stands for the "end site in SEQ ID NO: 2", and indicates the position number of the nucleoside on a side closest to a 5' side targeted by an antisense oligonucleotide in the human CALM2 pre-mRNA sequence.

Each antisense oligonucleotide targets any of the human CALM2 mRNA designated as SEQ ID NO: 1 in the present specification and/or the human CALM2 pre-mRNA designated as SEQ ID NO: 2 in the present specification.

"SEQ No" stands for SEQ ID NO, and "BASE SEQUENCE" stands for the nucleobase sequence of the antisense oligonucleotide.

**[Table2]**

| Compd No | SEQ1 START | SEQ1 END | SEQ2 START | SEQ2 END | SEQ No | BASE SEQUENCE |
|---|---|---|---|---|---|---|
| P19710001 | 620 | 635 | 15838 | 15853 | 3 | GGTCTTCACTTTGCTG |
| P19710002 | 907 | 922 | 16125 | 16140 | 4 | CCCAGATGTTCCCTCA |
| P19710003 | 160 | 175 | 160 | 175 | 5 | AAGCGCTACCGGTTTC |
| P19710004 | 1062 | 1077 | 16280 | 16295 | 6 | GTGTACGAGTACCTGG |
| P19710006 | 102 | 117 | 102 | 117 | 7 | GGACTAATTCGCCTCC |
| P19710007 | 995 | 1010 | 16213 | 16228 | 8 | GTCCATAGTCCACGCA |
| P19710008 | 1151 | 1166 | 16369 | 16384 | 9 | GCAGATTTTAAGTGGC |
| P19710009 | 183 | 198 | 5859 | 5874 | 10 | AGTCAGTTGGTCAGCC |
| P19710010 | 365 | 380 | 14238 | 14253 | 11 | GGGAAGTCAATTGTGC |
| P19710011 | 715 | 730 | 15933 | 15948 | 12 | TTTGACAGTAGGGAGA |
| P19710012 | 1239 | 1254 | 16457 | 16472 | 13 | GCAGAGCAACCATTGG |
| P19710014 | 322 | 337 | 14069 | 14084 | 14 | TCATGTCCTGTAACTC |
| P19710015 | 862 | 877 | 16080 | 16095 | 15 | GCAGAAGGGCTTAGAT |
| P19710016 | 1140 | 1155 | 16358 | 16373 | 16 | GTGGCTAAACAAAGTT |
| P19710017 | 212 | 227 | 13959 | 13974 | 17 | GCTTCTTTGAATTCTG |
| P19710018 | 479 | 494 | 14779 | 14794 | 18 | TCTGCAGCACTAATAT |
| P19710019 | 387 | 402 | 14260 | 14275 | 19 | TGCCATCATTGTCAGA |
| P19710020 | 1089 | 1104 | 16307 | 16322 | 20 | GTACAGGACCAGCAGT |

### [Evaluation Example 1] Antisense suppression of human CALM2 in HepG2 cells

HepG2 cells were seeded in a 96-well plate at a density of 10,000 cells/well, and after about 24 hours, the antisense oligonucleotides (produced in Production Example 1) were added thereto using Lipofectamine (registered trademark) 3000 (Thermo Fisher Scientific Inc.) so that the final concentrations of the antisense oligonucleotides were 50 nM (transfection). After 24 hours, RNAs were isolated from the cells using RNeasy Mini Kit (QIAGEN) and then reverse transcribed with PrimeScript (registered trademark) RT Master Mix (Perfect Real Time) (Takara Bio Inc.), thus preparing cDNAs. Using the prepared cDNAs, the CALM2 gene expression levels were measured by performing quantitative real-time PCR with TaqMan (registered trademark) Gene Expression Assays (Thermo Fisher Scientific Inc.). During the real-time PCR, the mRNA amount of the housekeeping gene peptidylprolyl isomerase A [PPIA] was simultaneously quantified, and the mRNA amounts of CALM2 relative to the mRNA amount of PPIA were evaluated as the CALM2 expression levels. The results are indicated in Table 3 as percent expression of CALM2 relative to the untreated control cells.

**[Table3]**

| Compd No | CALM2 mRNA remain % |
|---|---|
| P19710001 | 1 |
| P19710002 | 10 |
| P19710003 | 3 |
| P19710004 | 1 |
| P19710006 | 37 |
| P19710007 | 4 |
| P19710008 | 1 |
| P19710009 | 13 |
| P19710010 | 4 |
| P19710011 | 3 |
| P19710012 | 9 |
| P19710014 | 4 |
| P19710015 | 13 |
| P19710016 | 1 |
| P19710017 | 2 |
| P19710018 | 11 |
| P19710019 | 12 |
| P19710020 | 1 |

### [Evaluation Example 2] Dose-dependent antisense suppression of human CALM1/CALM2/CALM3 in HepG2 cells

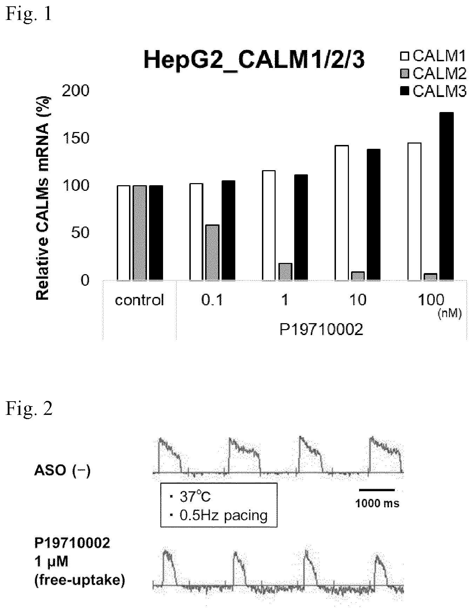

HepG2 cells were seeded in a 96-well plate at a density of 10,000 cells/well, and after about 24 hours, P19710002 was added thereto using Lipofectamine (registered trademark) 3000 (Thermo Fisher Scientific Inc.) so that the final concentrations of P19710002 were 0.1 nM, 1 nM, 10 nM, and 100 nM (transfection). After 24 hours, RNAs were isolated from the cells using RNeasy Mini Kit (QIAGEN) and then reverse transcribed with PrimeScript (registered trademark) RT Master Mix (Perfect Real Time) (Takara Bio Inc.), thus preparing cDNAs. Using the prepared cDNAs, each of the CALM1, CALM2, and CALM3 gene expression levels was measured by performing quantitative real-time PCR with TaqMan (registered trademark) Gene Expression Assays (Thermo Fisher Scientific Inc.). During the real-time PCR, the mRNA amount of the housekeeping gene PPIA was simultaneously quantified, and the mRNA amounts of CALM1, CALM2, and CALM3 relative to the mRNA amount of the PPIA were evaluated as the CALM1, CALM2, and CALM3 expression levels. The results are indicated in Fig. 1 as percent expression of CALM2 relative to the untreated control cells (control).

As is clear from Fig. 1, P19710002 suppressed the CALM2 expression in a dose-dependent manner. On the other hand, it was shown that P19710002 did not suppress the expression of the family genes CALM1 and CALM3.

### [Evaluation Example 3] Antisense suppression of mouse CALM2 in 3T3-L1 cells

3T3-L1 cells were seeded in a 96-well plate at a density of 10,000 cells/well, and after about 24 hours, P19710002 was added thereto so that the final concentrations of P19710002 were 0.1 nM, 1 nM, 10 nM, and 100 nM (free-uptake). After 5 days, RNAs were isolated from the cells using RNeasy Mini Kit (QIAGEN) and then reverse transcribed with PrimeScript (registered trademark) RT Master Mix (Perfect Real Time) (Takara Bio Inc.), thus preparing cDNAs. Using the prepared cDNAs, the mouse CALM2 gene expression levels were measured by performing quantitative real-time PCR with TaqMan (registered trademark) Gene Expression Assays (Thermo Fisher Scientific Inc.). During the real-time PCR, the mRNA amount of the housekeeping gene PPIA was simultaneously quantified, and the mRNA amounts of CALM2 relative to the mRNA amount of PPIA were evaluated as the CALM2 expression levels. The results are indicated in Table 4 as percent expression of CALM2 relative to the untreated control cells. Note that "concentration" in Table 4 indicates the concentration of P19710002.

**[Table4]**

| concentration (nM) | CALM2 mRNA remain % |
|---|---|
| 0.1 | 96 |
| 1 | 79 |
| 10 | 32 |
| 100 | 9 |

### [Reference Example 1] Establishment of LQT15 patient-derived iPS cells

Blood was collected from an LQT15 patient in which a genetic mutation in CALM2 (c.293A >G, p. N98S) was identified, and LQT15 patient-derived iPS cells were prepared in the same manner as in Non Patent Literature 4.

### [Reference Example 2] Preparation of cardiomyocytes differentiated from LQT15 patient-derived iPS cells

The iPS cells established in Reference Example 1 were differentiated into myocardium using a two-dimensional differentiation induction method referred to as the GiWi method (Lian et al, Nat Protoc. 2013 Jan; 8 (1): 162-75). iPS cells cultured on a plate coated with Matrigel Growth Factor Reduced (registered trademark) diluted using Iscove's Modified Dulbecco's Medium (IMDM) were cultured for one day in a medium obtained by adding B-27 (registered trademark) supplement to RPMI 1640 (insulin (-)) with added CHIR99021 [6-{2-[4-(2,4-dichloro-pheny)-5-(5-methyl-1H-imidazol-2-yl)-pyrimidin-2-ylamino]-ethylamino}-nicotinonitrile]. From the third day after the initiation of the differentiation, the cells were cultured with added IWP-2 [[N-(6-methyl-2-benzothiazolyl)-2-[(3,4,6,7-tetrahydro-4-oxo-3-phenylthieno[3,2-d]pyrimidin-2-yl)thio]-acetamide] for 2 days, and after the seventh day after the initiation of the differentiation, the culturing was performed in a medium obtained by adding B-27 (registered trademark) supplement and insulin to RPMI 1640. After 12 days, beating cardiomyocytes appeared.

### [Evaluation Example 4] Functional evaluation of cardiomyocytes differentiated from LQT15 patient-derived human iPS cells

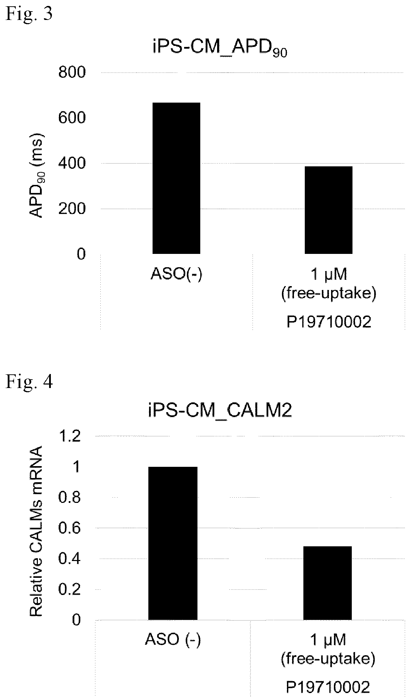

Sheet-shaped iPS cell-derived myocardium was prepared by suspending 50,000 cardiomyocytes differentiated in Reference Example 2 in 4 µL of a culture solution and seeding the suspended cardiomyocytes in a 96-well plate coated with Matrigel Growth Factor Reduced (registered trademark) diluted using 4 µL of Iscove's Modified Dulbecco's Medium (IMDM). After about 24 hours, P19710002 was added so that the final concentration thereof was 1 µM (free-uptake). After about 120 hours, loading was performed with a membrane potential-sensitive dye Fluovolt (Thermo Fisher Scientific Inc.) at 37°C for 30 minutes, and action potentials were measured at 37°C and 0.5 Hz pacing using an imaging system (microscope: Nikon Corporation, high-speed optical measurement system: Brainvision Inc.). The results are shown in Figs. 2 and 3. Note that action potentials in a case of not adding P19710002 were measured together, which are indicated in Figs. 2 and 3 as ASO (-). "ms" in Figs. 2 and 3 stands for milliseconds.

As is clear from Figs. 2 and 3, P19710002 which is an antisense oligonucleotide of CALM2 is shown to shorten the 90% action potential duration (APD₉₀).

### [Evaluation Example 5] Antisense suppression of human CALM2 in cardiomyocytes differentiated from LQT15 patient-derived human iPS cells

The cardiomyocytes differentiated in Reference Example 2 were seeded in a 96-well plate at a density of 200,000 cells per well. After about 24 hours, P19710002 was added so that the final concentration thereof was 1 µM (free-uptake). After about 120 hours, RNAs were extracted from the cells using NucleoSpin RNA Plus XS (Takara Bio Inc.) and then reverse transcribed with Transcriptor First Strand cDNA Synthesis Kit (F. Hoffmann-La Roche Ltd), thus preparing cDNAs. Using the prepared cDNAs, the mouse CALM2 gene expression level was measured by performing quantitative real-time PCR by the TaqMan method. During the real-time PCR, the mRNA amount of the housekeeping gene Glyceraldehyde-3-Phosphate Dehydrogenase (GAPDH) was simultaneously quantified, and the mRNA amount of CALM2 relative to the mRNA amount of GAPDH was evaluated as the CALM2 expression level. The results are shown in Fig. 4. Note that ASO (-) indicates a case where P19710002 was not added.

### [Production Example 2]

The antisense oligonucleotide indicated in Table 5 (compound represented by the chemical structure corresponding to the compound number) was prepared using an automated nucleic acid synthesizer nS-8II (manufactured by GeneDesign, Inc.). In the sequence indicated in Table 5, "C16-" indicates that the moiety at the 5' terminal in which the hydrogen atom is removed from the hydroxy group is bonded to the group represented by the following Formula (I). 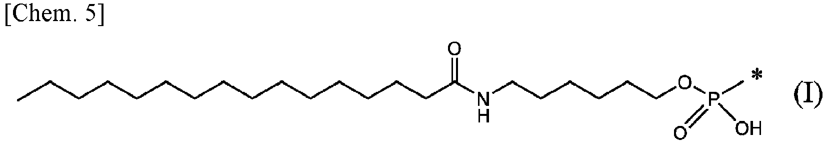 (In the formula, ^{∗} represents the position of the bonding to the oligonucleotide) Note that the nucleobase sequence of P19710036 is SEQ ID NO: 4.

**[Table5]**

| Compd No | Chemical Structure |
|---|---|
| P19710036 | C16-5(L)^5(L)^5(L)^a^g^a^t^g^t^t^5(x)^5(x)^5(x)^T(L)^5(L)^A(L) |

### [Evaluation Example 6] Antisense suppression of CALM2 in mice

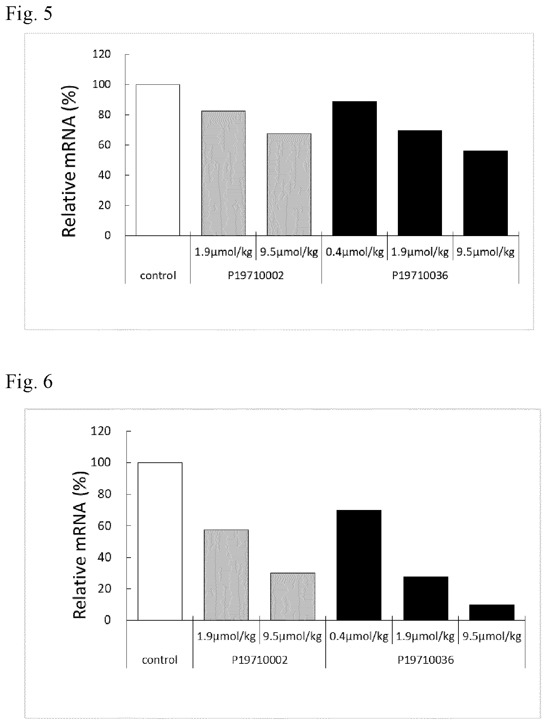

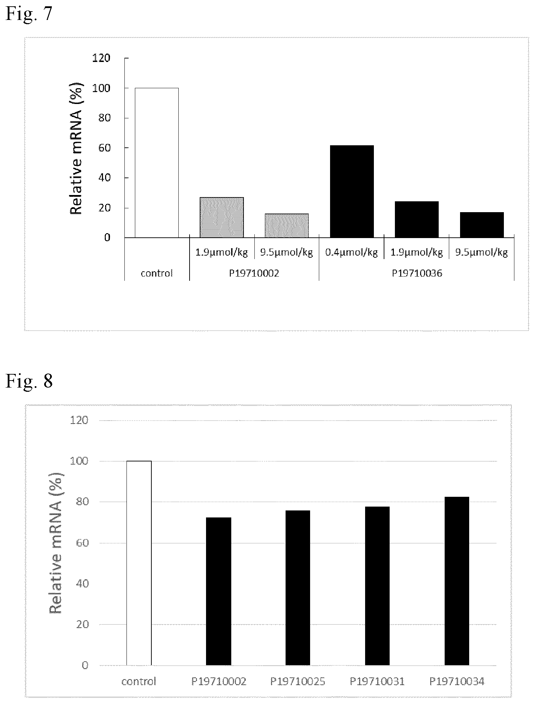

P19710002 and P19710036 dissolved in physiological saline (Otsuka Normal Saline, Otsuka Pharmaceutical Factory, Inc.) were intravenously administered to C57BL/6J mice (male, 6 weeks old, Charles River Laboratories Japan, Inc.) so that the dose per individual mouse was 1.9 µmol/kg or 9.5 µmol/kg in terms of the amount of the antisense oligonucleotide. Intravenous administration of only P19710036 was also performed at a dose of 0.4 µmol/kg. As a control, only physiological saline (Otsuka Normal Saline, Otsuka Pharmaceutical Factory, Inc.) was administered. 5 days after the administration, heart, liver, and kidney tissues were collected under isoflurane anesthesia. RNAs were isolated from each of the organs using RNeasy Mini Kit (manufactured by QIAGEN) and then cDNAs were obtained using PrimeScript (registered trademark) RT Master Mix (Perfect Real Time) (Takara Bio Inc.). Using the prepared cDNAs, the mouse CALM2 gene expression levels were measured by performing quantitative real-time PCR with TaqMan (registered trademark) Gene Expression Assays (Thermo Fisher Scientific Inc.). During the real-time PCR, the mRNA amount of the housekeeping gene PPIA was simultaneously quantified, and the mRNA amounts of CALM2 relative to the mRNA amount of PPIA were evaluated as the CALM2 expression levels. The results are indicated in Fig. 5 (heart), Fig. 6 (liver), and Fig. 7 (kidney) as percent expression of CALM2 relative to the untreated control group (control).

As is clear from Figs. 5 to 7, P19710002 and P19710036 suppressed the expression of CALM2 in the heart, liver, and kidney.

### [Production Example 3]

The antisense oligonucleotides indicated in Table 6 (compounds represented by the chemical structures corresponding to the compound numbers) were prepared using an automated nucleic acid synthesizer nS-8II (manufactured by GeneDesign, Inc.).

**[Table6]**

| Compd No | Chemical Structure |
|---|---|
| P19710021 | 5(V)^5(L)^T(L)^5(x)^a^t^t^t^g^a^5(x)^5(x)^a^A(L)^5(L)^T(V) |
| P19710022 | T(V)^5(L)^5(L)^5(x)^t^5(x)^a^t^t^t^g^a^5(x)^5(L)^A(L)^A(V) |
| P19710023 | G(V)^T(L)^T(L)^5(x)^5(x)^5(x)^t^5(x)^a^t^t^t^g^A(L)^5(L)^5(V) |
| P19710024 | 5(V)^5(L)^A(L)^g^a^t^g^t^t^5(x)^5(x)^5(x)^t^5(L)^A(L)^T(V) |
| P19710025 | A(V)^5(L)^5(L)^5(x)^a^g^a^t^g^t^t^5(x)^5(x)^5(L)^T(L)^5(V) |
| P19710026 | A(V)^A(L)^5(L)^5(x)^5(x)^a^g^a^t^g^t^t^5(x)^5(L)^5(L)^T(V) |
| P19710027 | T(V)^A(L)^A(L)^5(x)^5(x)^5(x)^a^g^a^t^g^t^t^5(L)^5(L)^5(V) |
| P19710028 | A(V)^G(L)^G(L)^5(x)^a^t^a^a^5(x)^5(x)^5(x)^a^g^A(L)^T(L)^G(V) |
| P19710029 | A(V)^A(L)^A(L)^g^g^5(x)^a^t^a^a^5(x)^5(x)^5(x)^A(L)^G(L)^A(V) |
| P19710030 | A(V)^A(L)^A(L)^a^a^g^g^5(x)^a^t^a^a^5(x)^5(L)^5(L)^A(V) |
| P19710031 | 5(V)^5(L)^5(L)^a^g^a^t^g^t^t^5(x)^5(x)^5(x)^T(L)^5(L)^A(V) |
| P19710032 | 5(V)^5(V)^5(L)^a^g^a^t^g^t^t^5(x)^5(x)^5(x)^T(L)^5(L)^A(V) |
| P19710033 | 5(V)^5(L)^5(L)^a^g^a^t^g^t^t^5(x)^5(x)^5(x)^T(L)^5(V)^A(V) |
| P19710034 | 5(L)^5(V)^5(L)^a^g^a^t^g^t^t^5(x)^5(x)^5(x)^T(L)^5(V)^A(L) |
| P19710035 | A(V)^5(L)^5(V)^5(L)^a^g^a^t^g^t^t^5(x)^5(x)^5(x)^T(L)^5(V)^A(L)^T(V) |

Positions in the human CALM2 mRNA or pre-mRNA sequence targeted by each of the antisense oligonucleotides indicated in Table 6 and SEQ ID NO and the nucleobase sequence corresponding to each of the antisense oligonucleotides are indicated in Table 7. Note that the notations in Table 7 are the same as those in Table 2.

**[Table7]**

| Compd No | SEQ1 START | SEQ1 END | SEQ2 START | SEQ2 END | SEQ No | BASE SEQUENCE |
|---|---|---|---|---|---|---|
| P19710021 | 896 | 911 | 16114 | 16129 | 21 | CCTCATTTGACCAACT |
| P19710022 | 898 | 913 | 16116 | 16131 | 22 | TCCCTCATTTGACCAA |
| P19710023 | 900 | 915 | 16118 | 16133 | 23 | GTTCCCTCATTTGACC |
| P19710024 | 906 | 921 | 16124 | 16139 | 24 | CCAGATGTTCCCTCAT |
| P19710025 | 908 | 923 | 16126 | 16141 | 25 | ACCCAGATGTTCCCTC |
| P19710026 | 909 | 924 | 16127 | 16142 | 26 | AACCCAGATGTTCCCT |
| P19710027 | 910 | 925 | 16128 | 16143 | 27 | TAACCCAGATGTTCCC |
| P19710028 | 915 | 930 | 16133 | 16148 | 28 | AGGCATAACCCAGATG |
| P19710029 | 917 | 932 | 16135 | 16150 | 29 | AAAGGCATAACCCAGA |
| P19710030 | 919 | 934 | 16137 | 16152 | 30 | AAAAAGGCATAACCCA |
| P19710031 | 907 | 922 | 16125 | 16140 | 4 | CCCAGATGTTCCCTCA |
| P19710032 | 907 | 922 | 16125 | 16140 | 4 | CCCAGATGTTCCCTCA |
| P19710033 | 907 | 922 | 16125 | 16140 | 4 | CCCAGATGTTCCCTCA |
| P19710034 | 907 | 922 | 16125 | 16140 | 4 | CCCAGATGTTCCCTCA |
| P19710035 | 907 | 922 | 16125 | 16140 | 4 | CCCAGATGTTCCCTCA |

### [Evaluation Example 7] Antisense suppression of human CALM2 in HepG2 cells

Using the same evaluation method as in Evaluation Example 1, the antisense oligonucleotides (produced in Production Example 3) were added so that the final concentrations thereof were 30 nM, and the mRNA amounts of CALM2 relative to the mRNA amount of PPIA were evaluated as the CALM2 expression levels. The results are indicated in Table 8 as percent expression of CALM2 relative to the untreated control cells.

**[Table8]**

| Compd No | CALM2 mRNA remain % |
|---|---|
| P19710021 | 29 |
| P19710022 | 14 |
| P19710024 | 12 |
| P19710025 | 15 |
| P19710026 | 16 |
| P19710027 | 16 |
| P19710029 | 13 |
| P19710030 | 15 |
| P19710031 | 14 |
| P19710032 | 21 |
| P19710033 | 17 |
| P19710034 | 17 |
| P19710035 | 22 |
| P19710023 | 19 |
| P19710028 | 6 |

### [Evaluation Example 8] Antisense suppression of human CALM2 in HepG2 cells

The experiment was conducted using the same evaluation method as in Evaluation Example 1 at various concentrations of antisense oligonucleotides (produced in Production Examples 1 and 3). The antisense oligonucleotides were added so that the final concentrations thereof were 0.3 nM, 1 nM, and 30 nM, and the mRNA amounts of CALM2 relative to the mRNA amount of PPIA were evaluated as the CALM2 expression levels. The results were calculated as concentrations at which the RNA transcript level was 50% relative to the untreated control cells (IC50 value), which are indicated in Table 9.

**[Table9]**

| Compd No | IC₅₀ (nM) |
|---|---|
| P19710002 | 0.8 |
| P19710025 | 1.4 |
| P19710031 | 1.0 |
| P19710032 | 2.8 |
| P19710033 | 2.5 |
| P19710034 | 1.5 |
| P19710035 | 2.2 |
| P19710023 | 5.2 |
| P19710028 | 1.9 |

### [Evaluation Example 9] Antisense suppression of CALM2 in mice

The same evaluation method as in Evaluation Example 6 was used. P19710002, P19710025, P19710031, and P19710034 were intravenously administered to mice so that the dose per individual mouse was 9.5 µmol/kg in terms of the amount of the antisense oligonucleotide. As a control, only physiological saline (Otsuka Normal Saline, Otsuka Pharmaceutical Factory, Inc.) was administered. The mRNA amounts of CALM2 relative to the mRNA amount of PPIA in the heart tissue 5 days after the administration were evaluated as the CALM2 expression levels. The results are indicated in Fig. 8 as percent expression of CALM2 relative to the untreated control group (control).

As is clear from Fig. 8, P19710002, P19710025, P19710031, and P19710034 suppressed the expression of CALM2 in the heart.

### [Production Example 4]

The antisense oligonucleotides indicated in Table 10 (compounds represented by the chemical structures corresponding to the compound numbers) were prepared using an automated nucleic acid synthesizer nS-8II (manufactured by GeneDesign, Inc.).

**[Table10]**

| Compd No | Chemical Structure |
|---|---|
| P19710039 | A(L)^5(L)^T(L)^5(x)^a^5(x)^5(x)^a^t^g^5(x)^t^g^5(L)^A(L)^A(L) |
| P19710040 | A(L)^A(L)^G(L)^a^t^g^t^5(x)^a^5(x)^5(x)^t^t^G(L)^5(L)^5(L) |
| P19710041 | G(L)^G(L)^A(L)^g^g^t^t^t^5(x)^a^g^t^t^G(L)^G(L)^5(L) |
| P19710042 | A(L)^G(L)^T(L)^g^5(x)^5(x)^a^t^t^t^t^5(x)^t^T(L)^A(L)^G(L) |
| P19710043 | 5(L)^A(L)^G(L)^t^5(x)^a^g^t^t^g^g^t^5(x)^A(L)^G(L)^5(L) |
| P19710044 | G(L)^5(L)^T(L)^5(x)^t^t^5(x)^a^g^t^5(x)^a^g^T(L)^T(L)^G(L) |
| P19710045 | A(L)^5(L)^5(L)^t^g^5(x)^a^a^t^5(x)^t^g^5(x)^T(L)^5(L)^T(L) |
| P19710046 | T(L)^T(L)^G(L)^t^g^5(x)^5(x)^a^t^t^a^5(x)^5(x)^T(L)^G(L)^A(L) |
| P19710047 | G(L)^T(L)^5(L)^a^g^a^a^a^t^t^5(x)^a^g^G(L)^G(L)^A(L) |
| P19710048 | 5(L)^5(L)^A(L)^t^5(x)^a^t^t^g^t^5(x)^a^g^A(L)^A(L)^A(L) |
| P19710049 | T(L)^5(L)^T(L)^t^g^5(x)^5(x)^a^t^5(x)^a^t^t^G(L)^T(L)^5(L) |
| P19710050 | A(L)^5(L)^T(L)^g^t^5(x)^t^g^t^g^t^5(x)^t^T(L)^T(L)^5(L) |
| P19710051 | T(L)^5(L)^T(L)^t^5(x)^t^t^5(x)^a^5(x)^t^g^t^5(L)^T(L)^G(L) |
| P19710052 | T(L)^A(L)^G(L)^5(x)^5(x)^a^t^t^g^5(x)^5(x)^a^t^5(L)^5(L)^T(L) |
| P19710053 | G(L)^5(L)^A(L)^5(x)^t^a^a^t^a^t^a^g5(x)^5(L)^A(L)^T(L) |
| P19710054 | 5(L)^5(L)^A(L)^a^g^g^t^t^t^g^t^5(x)^a^T(L)^5(L)^A(L) |
| P19710055 | T(L)^5(L)^T(L)^t^5(x)^a^t^5(x)^t^g^t^t^a^A(L)^5(L)^T(L) |
| P19710056 | T(L)^G(L)^T(L)^5(x)^a^t^5(x)^a^t^t^t^g^t^A(L)^5(L)^A(L) |
| P19710057 | A(L)^A(L)^5(L)^a^5(x)^a^t^t^5(x)^t^g^t^a^5(L)^A(L)^A(L) |
| P19710058 | T(L)^A(L)^5(L)^a^g^a^t^a^a^g^t^t^a^5(L)^A(L)^A(L) |
| P19710059 | T(L)^T(L)^T(L)^t^t^t^g^a^5(x)^a^g^t^a^G(L)^G(L)^G(L) |
| P19710060 | T(L)^A(L)^5(L)^t^a^t^a^5(x)^a^t^g^5(x)^a^T(L)^A(L)^T(L) |
| P19710061 | T(L)^G(L)^G(L)^a^g^g^a^a^t^g^a^a^g^T(L)^5(L)^5(L) |
| P19710062 | G(L)^G(L)^G(L)^a^a^g^a^a^a^a^5(x)^a^t^G(L)^G(L)^A(L) |
| P19710063 | G^(L)^T(L)^A(L)^a^g^a^t^a^a^g^g^g^a^A(L)^G(L)^A(L) |
| P19710064 | G(L)^A(L)^5(L)^a^g^t^a^a^g^a^t^a^a^G(L)^G(L)^G(L) |
| P19710065 | G(L)^G(L)^A(L)^5(x)^a^a^t^g^a^5(x)^a^gt^A(L)^A(L)^G(L) |
| P19710066 | 5(L)^A(L)^T(L)^g^5(x)^a^a^5(x)^a^t^g^t^t^A(L)^5(L)^T(L) |
| P19710067 | A(L)^G(L)^5(L)^6(x)^a^5(x)^a^t^g^5(x)^a^a^5(x)^A(L)^T(L)^G(L) |
| P19710068 | 5(L)^5(L)^A(L)^g^a^g^t^a^a^g^5(x)^5(x)^a^5(L)^A(L)^T(L) |
| P19710069 | A(L)^G(L)^T(L)^t^t^a^g^a^t^g^t^g^5(x)^A(L)^G(L)^A(L) |
| P19710070 | 5(L)^A(L)^A(L)^5(x)^t^5(x)^5(x)^a^t^5(x)^t^a^a^G(L)^T(L)^T(L) |
| P19710071 | 5(L)^A(L)^T(L)^g^5(x)^t^g^a^5(x)^a^g^t^t^5(L)^5(L)^T(L) |
| P19710072 | A(L)^5(L)^A(L)^a^5(x)^a^t^g^5(x)^t^g^a^5(x)^A(L)^G(L)^T(L) |
| P19710073 | T(L)^A(L)^T(L)^t^g^t^t^g^a^5(x)^t^g^t5(L)^5(L)^A(L) |
| P19710074 | 5(L)^G(L)^T(L)^t^t^t^g^5(x)^a^a^t^a^g^T(L)^G(L)^5(L) |
| P19710075 | 5(L)^5(L)^A(L)^g^5(x)^a^g^t^a^5(x)^a^a^a^A(L)^A(L)^A(L) |
| P19710076 | G(L)^G(L)^5(L)^t^a^a^a^5(x)^a^a^a^g^t^T(L)^T(L)^A(L) |
| P19710077 | A(L)^A(L)^G(L)^t^g^g^5(x)^t^a^a^a^5(x)^a^A(L)^A(L)^G(L) |
| P19710078 | T(L)^T(L)^T(L)^a^a^g^t^g^g^5(x)^t^a^a^A(L)^5(L)^A(L) |
| P19710079 | 5(L)^A(L)^A(L)^5(x)^t^t^g^g^a^a^t^g^g^A(L)^T(L)^T(L) |
| P19710005 | 5(L)^T(L)^5(L)^a^t^a^a^a^5(x)^a^5(x)^5(x)^t^5(L)^5(L)^5(L) |
| P19710013 | 5^(L)^5(L)^5(L)^a^g^5(x)^g^5(x)^5(x)^t^5(x)^a^t^A(L)^A(L)^A(L) |

Positions in the human CALM2 mRNA or pre-mRNA sequence targeted by each of the antisense oligonucleotides indicated in Table 10 and SEQ ID NO and the nucleobase sequence corresponding to each of the antisense oligonucleotides are indicated in Table 11.

"-" (hyphen) indicates that the antisense oligonucleotide does not target the mRNA or pre-mRNA sequence at 100% complementarity. Other notations in Table 11 are the same as those in Table 2.

**[Table11]**

| Compd No | SEQ1 START | SEQ1 END | SEQ2 START | SEQ2 END | SEQ No | BASE SEQUENCE |
|---|---|---|---|---|---|---|
| P19710039 | - | - | 174 | 189 | 31 | ACTCACCATGCTGCAA |
| P19710040 | - | - | 1624 | 1639 | 32 | AAGATGTCACCTTGCC |
| P19710041 | - | - | 1661 | 1676 | 33 | GGAGGTTTCAGTTGGC |
| P19710042 | - | - | 3355 | 3370 | 34 | AGTGCCATTTTCTTAG |
| P19710043 | 184 | 199 | 5860 | 5875 | 35 | CAGTCAGTTGGTCAGC |
| P19710044 | 190 | 205 | 5866 | 5881 | 36 | GCTCTTCAGTCAGTTG |
| P19710045 | - | - | 5877 | 5892 | 37 | ACCTGCAATCTGCTCT |
| P19710046 | - | - | 14228 | 14243 | 38 | TTGTGCCATTACCTGA |
| P19710047 | 377 | 392 | 14250 | 14265 | 39 | GTCAGAAATTCAGGGA |
| P19710048 | 385 | 400 | 14258 | 14273 | 40 | CCATCATTGTCAGAAA |
| P19710049 | 390 | 405 | 14263 | 14278 | 41 | TCTTGCCATCATTGTC |
| P19710050 | 411 | 426 | 14284 | 14299 | 42 | ACTGTCTGTGTCTTTC |
| P19710051 | 419 | 434 | 14292 | 14307 | 43 | TCTTCTTCACTGTCTG |
| P19710052 | 464 | 479 | 14764 | 14779 | 44 | TAGCCATTGCCATCCT |
| P19710053 | 473 | 488 | 14773 | 14788 | 45 | GCACTAATATAGCCAT |
| P19710054 | 506 | 521 | 14806 | 14821 | 46 | CCAAGGTTTGTCATCA |
| P19710055 | 527 | 542 | 14827 | 14842 | 47 | TCTTCATCTGTTAACT |
| P19710056 | 606 | 621 | 15824 | 15839 | 48 | TGTCATCATTTGTACA |
| P19710057 | 636 | 651 | 15854 | 15869 | 49 | AACACATTCTGTACAA |
| P19710058 | 692 | 707 | 15910 | 15925 | 50 | TACAGATAAGTTACAA |
| P19710059 | 718 | 733 | 15936 | 15951 | 51 | TTTTTTGACAGTAGGG |
| P19710060 | 734 | 749 | 15952 | 15967 | 52 | TACTATACATGCATAT |
| P19710061 | 754 | 769 | 15972 | 15987 | 53 | TGGAGGAATGAAGTCC |
| P19710062 | 766 | 781 | 15984 | 15999 | 54 | GGGAAGAAAACATGGA |
| P19710063 | 775 | 790 | 15993 | 16008 | 55 | GTAAGATAAGGGAAGA |
| P19710064 | 779 | 794 | 15997 | 16012 | 56 | GACAGTAAGATAAGGG |
| P19710065 | 786 | 801 | 16004 | 16019 | 57 | GGACAATGACAGTAAG |
| P19710066 | 829 | 844 | 16047 | 16062 | 58 | CATGCAACATGTTACT |
| P19710067 | 834 | 849 | 16052 | 16067 | 59 | AGCCACATGCAACATG |
| P19710068 | 842 | 857 | 16060 | 16075 | 60 | CCAGAGTAAGCCACAT |
| P19710069 | 873 | 888 | 16091 | 16106 | 61 | AGTTTAGATGTGCAGA |
| P19710070 | 885 | 900 | 16103 | 16118 | 62 | CAACTCCATCTAAGTT |
| P19710071 | 951 | 966 | 16169 | 16184 | 63 | CATGCTGACAGTTCCT |
| P19710072 | 955 | 970 | 16173 | 16188 | 64 | ACAACATGCTGACAGT |
| P19710073 | 1006 | 1021 | 16224 | 16239 | 65 | TATTGTTGACTGTCCA |
| P19710074 | 1036 | 1051 | 16254 | 16269 | 66 | CGTTTTGCAATAGTGC |
| P19710075 | 1081 | 1096 | 16299 | 16314 | 67 | CCAGCAGTACAAAAAA |
| P19710076 | 1138 | 1153 | 16356 | 16371 | 68 | GGCTAAACAAAGTTTA |
| P19710077 | 1142 | 1157 | 16360 | 16375 | 69 | AAGTGGCTAAACAAAG |
| P19710078 | 1145 | 1160 | 16363 | 16378 | 70 | TTTAAGTGGCTAAACA |
| P19710079 | 1188 | 1203 | 16406 | 16421 | 71 | CAACTTGGAATGGATT |
| P19710005 | 72 | 87 | 72 | 87 | 72 | CTCATAAACACCTCCC |
| P19710013 | 80 | 95 | 80 | 95 | 73 | CCCAGCGCCTCATAAA |

### [Evaluation Example 10] Antisense suppression of human CALM2 in HepG2 cells

Using the same evaluation method as in Evaluation Example 1, the antisense oligonucleotides (produced in Production Example 4) were added so that the final concentrations thereof were 10 nM, and the mRNA amounts of CALM2 relative to the mRNA amount of PPIA were evaluated as the CALM2 expression levels. The results are indicated in Table 12 as percent expression of CALM2 relative to the untreated control cells.

**[Table12]**

| Compd No | CALM2 mRNA remain % |
|---|---|
| P19710040 | 13 |
| P19710041 | 6 |
| P19710042 | 18 |
| P19710043 | 21 |
| P19710044 | 21 |
| P19710046 | 35 |
| P19710047 | 45 |
| P19710048 | 35 |
| P19710049 | 47 |
| P19710050 | 9 |
| P19710051 | 36 |
| P19710052 | 34 |
| P19710053 | 18 |
| P19710054 | 18 |
| P19710056 | 77 |
| P19710057 | 39 |
| P19710058 | 62 |
| P19710059 | 31 |
| P19710060 | 37 |
| P19710061 | 26 |
| P19710062 | 17 |
| P19710063 | 28 |
| P19710064 | 34 |
| P19710065 | 9 |
| P19710066 | 7 |
| P19710067 | 14 |
| P19710068 | 14 |
| P19710069 | 7 |
| P19710070 | 38 |
| P19710071 | 3 |
| P19710072 | 8 |
| P19710073 | 4 |
| P19710074 | 4 |
| P19710075 | 64 |
| P19710076 | 3 |
| P19710077 | 12 |
| P19710078 | 10 |
| P19710079 | 13 |

### [Production Example 5]

The antisense oligonucleotide indicated in Table 13 (compound represented by the chemical structure corresponding to the compound number) was prepared using an automated nucleic acid synthesizer nS-8II (manufactured by GeneDesign, Inc.). In the sequence indicated in Table 13, "Toc-TEG-" indicates that the moiety at the 5' terminal in which the hydrogen atom is removed from the hydroxy group is bonded to the group represented by the following Formula (II). 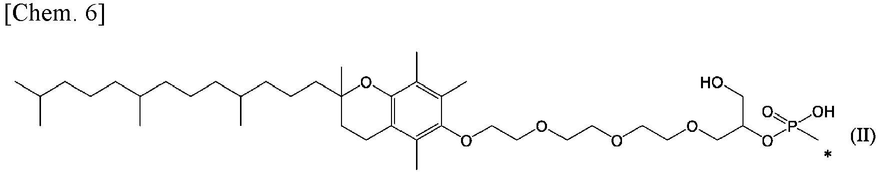 (In the formula, ^{∗} represents the position of the bonding to the oligonucleotide, the tocopherol moiety is in the DL form, the carbon atom to which the hydroxymethyl group is bonded is a mixture of the (R) form and the (S) form) Note that the nucleobase sequence of P20710091 is SEQ ID NO: 4.

**[Table13]**

| Compd No | Chemical Structure |
|---|---|
| P20710091 | Toc-TEG-5(L)^5(L)^5(L)^a^g^a^t^g^t^t^5(x)^5(x)^5(x)^T(L)^5(L)^A(L) |

### [Evaluation Example 11] Antisense suppression of CALM2 in mice

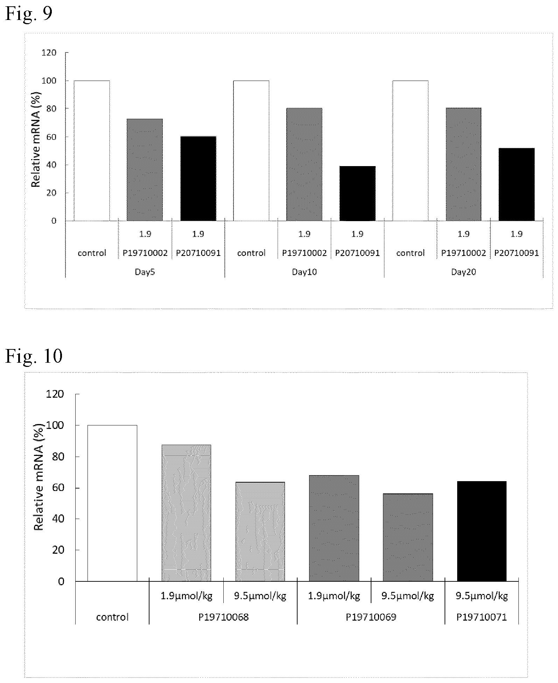

The same evaluation method as in Evaluation Example 6 was used. P19710002 and P20710091 were intravenously administered to mice so that the dose per individual mouse was 1.9 µmol/kg in terms of the amount of the antisense oligonucleotide. As a control, only physiological saline (Otsuka Normal Saline, Otsuka Pharmaceutical Factory, Inc.) was administered. The mRNA amounts of CALM2 relative to the mRNA amount of PPIA in the heart tissue 5 days, 10 days, and 20 days after the administration were evaluated as the CALM2 expression levels. The results are indicated in Fig. 9 as percent expression of CALM2 relative to the untreated control group (control).

As is clear from Fig. 9, P19710002 and P20710091 suppressed the expression of CALM2 in the heart.

### [Evaluation Example 12] Antisense suppression of CALM2 in mice

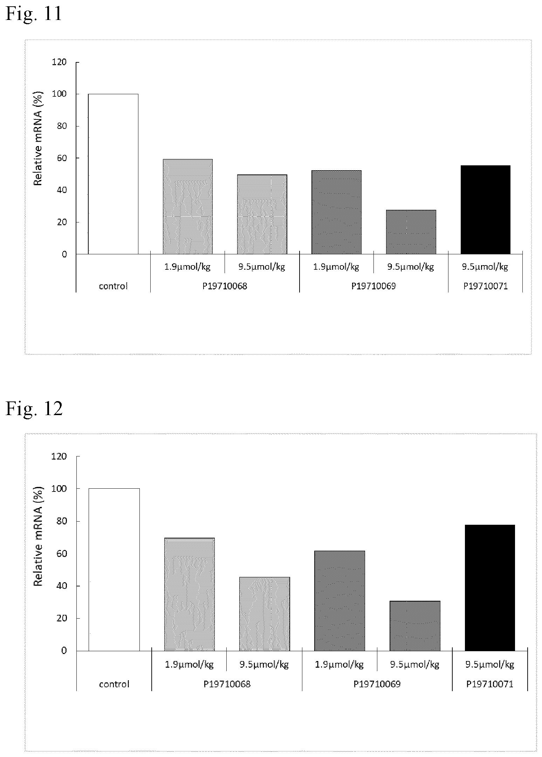

The same evaluation method as in Evaluation Example 6 was used. P19710068, P19710069, and P19710071 were intravenously administered to mice so that the dose per individual mouse was 1.9 µmol/kg or 9.5 µmol/kg in terms of the amount of the antisense oligonucleotide. As a control, only physiological saline (Otsuka Normal Saline, Otsuka Pharmaceutical Factory, Inc.) was administered. The mRNA amounts of CALM2 relative to the mRNA amount of PPIA in the heart, liver, and kidney tissues 5 days after the administration were evaluated as the CALM2 expression levels. The results are indicated in Fig. 10 (heart), Fig. 11 (liver), and Fig. 12 (kidney) as percent expression of CALM2 relative to the untreated control group (control).

As is clear from Figs. 10 to 12, P19710068, P19710069, and P19710071 suppressed the expression of CALM2 in the heart, liver, and kidney.

All publications or parts thereof cited herein are incorporated herein in their entirety by reference.

### Industrial Applicability

Since the compound containing the modified oligonucleotide in the present application or a pharmaceutically acceptable salt thereof can inhibit the expression of the CALM2 gene, the compound or a pharmaceutically acceptable salt thereof can be useful in treating preventing, and/or ameliorating a disease or a condition against which the inhibition of the CALM2 gene expression is effective, particularly, congenital long QT syndrome and even calmodulinopathy.

The present application is based on Japanese Patent Application No. 2020-036715 and Japanese Patent Application No. 2020-138019 filed in Japan, the entire contents of which are incorporated herein by reference.

## Claims

1. A compound or a pharmaceutically acceptable salt thereof comprising: a modified oligonucleotide with a length of 8 to 80 consecutive nucleosides, wherein the modified oligonucleotide has a nucleobase sequence containing at least 8 consecutive nucleobases contained in a nucleobase sequence of any one of SEQ ID NOs: 3 to 73.

2. The compound or a pharmaceutically acceptable salt thereof according to claim 1, wherein the modified oligonucleotide has a nucleobase sequence containing a nucleobase sequence of any one of SEQ ID NOs: 3 to 73.

3. The compound or a pharmaceutically acceptable salt thereof according to claim 1 or 2, wherein the modified oligonucleotide has a nucleobase sequence consisting of a nucleobase sequence of any one of SEQ ID NOs: 3 to 73.

4. A compound or a pharmaceutically acceptable salt thereof comprising: a modified oligonucleotide with a length of 8 to 80 consecutive nucleosides which is complementary to at least a part of a nucleobase sequence selected from the group consisting of the nucleobase sequences indicated by nucleobase position nos. 102 to 117, 160 to 175, 183 to 205, 212 to 227, 322 to 337, 365 to 405, 411 to 434, 464 to 494, 506 to 521, 606 to 635, 636 to 651, 692 to 707, 715 to 749, 754 to 801, 829 to 857, 862 to 934, 951 to 970, 995 to 1010, 1006 to 1021, 1036 to 1051, 1062 to 1077, 1081 to 1104, 1138 to 1166, 1188 to 1203, and 1239 to 1254 in SEQ ID NO: 1, wherein the modified oligonucleotide has at least 80% complementarity to at least a part of the nucleobase sequence in SEQ ID NO: 1.

5. A compound or a pharmaceutically acceptable salt thereof comprising: a modified oligonucleotide with a length of 8 to 80 consecutive nucleosides which is complementary to at least a part of a nucleobase sequence selected from the group consisting of the nucleobase sequences indicated by nucleobase position nos. 102 to 117, 160 to 175, 183 to 198, 212 to 227, 322 to 337, 365 to 380, 387 to 402, 479 to 494, 620 to 635, 715 to 730, 862 to 877, 896 to 934, 995 to 1010, 1062 to 1077, 1089 to 1104, 1140 to 1166, and 1239 to 1254 in SEQ ID NO: 1, wherein the modified oligonucleotide has at least 80% complementarity to at least a part of the nucleobase sequence in SEQ ID NO: 1.

6. A compound or a pharmaceutically acceptable salt thereof comprising: a modified oligonucleotide with a length of 8 to 80 consecutive nucleosides which is complementary to at least a part of the nucleobase sequence indicated by nucleobase position nos. 896 to 934, preferably, 907 to 922, in SEQ ID NO: 1, wherein the modified oligonucleotide has at least 80% complementarity to at least a part of the nucleobase sequence in SEQ ID NO: 1.

7. A compound or a pharmaceutically acceptable salt thereof comprising: a modified oligonucleotide with a length of 8 to 80 consecutive nucleosides which is complementary to at least a part of a nucleobase sequence selected from the group consisting of the nucleobase sequences indicated by nucleobase position nos. 102 to 117, 160 to 175, 1624 to 1639, 1661 to 1676, 3355 to 3370, 5859 to 5881, 13959 to 13974, 14069 to 14084, 14228 to 14278, 14284 to 14307, 14764 to 14794, 14806 to 14821, 15824 to 15869, 15910 to 15925, 15933 to 15967, 15972 to 16019, 16047 to 16075, 16080 to 16152, 16169 to 16188, 16213 to 16239, 16254 to 16269, 16280 to 16295, 16299 to 16322, 16356 to 16384, 16406 to 16421, and 16457 to 16472 in SEQ ID NO: 2, wherein the modified oligonucleotide has at least 80% complementarity to at least a part of the nucleobase sequence in SEQ ID NO: 2.

8. A compound or a pharmaceutically acceptable salt thereof comprising: a modified oligonucleotide with a length of 8 to 80 consecutive nucleosides which is complementary to at least a part of a nucleobase sequence selected from the group consisting of the nucleobase sequences indicated by nucleobase position nos. 102 to 117, 160 to 175, 5859 to 5874, 13959 to 13974, 14069 to 14084, 14238 to 14253, 14260 to 14275, 14779 to 14794, 15838 to 15853, 15933 to 15948, 16080 to 16095, 16114 to 16152, 16213 to 16228, 16280 to 16295, 16307 to 16322, 16358 to 16384, and 16457 to 16472 in SEQ ID NO: 2, wherein the modified oligonucleotide has at least 80% complementarity to at least a part of the nucleobase sequence in SEQ ID NO: 2.

9. A compound or a pharmaceutically acceptable salt thereof comprising: a modified oligonucleotide with a length of 8 to 80 consecutive nucleosides which is complementary to at least a part of the nucleobase sequence indicated by nucleobase position nos. 16114 to 16152, preferably, 16125 to 16140, in SEQ ID NO: 2, wherein the modified oligonucleotide has at least 80% complementarity to at least a part of the nucleobase sequence in SEQ ID NO: 2.

10. The compound or a pharmaceutically acceptable salt thereof according to any one of claims 1 to 9, wherein the modified oligonucleotide contains a phosphorothioate bond.

11. The compound or a pharmaceutically acceptable salt thereof according to any one of claims 1 to 10, wherein the modified oligonucleotide contains at least one selected from the group consisting of a 2'-modified nucleoside and a 2'-4'-bridged nucleoside.

12. The compound or a pharmaceutically acceptable salt thereof according to claim 11, wherein the 2'-4'-bridged nucleoside is at least one selected from the group consisting of an LNA, an ENA, a cEt, a BNA^{NC}, an AmNA, an scpBNA, and a GuNA.

13. The compound or a pharmaceutically acceptable salt thereof according to claim 12, wherein the 2'-4'-bridged nucleoside is an LNA.

14. The compound or a pharmaceutically acceptable salt thereof according to any one of claims 11 to 13, wherein the 2'-modified nucleoside is at least one selected from the group consisting of a 2'-O-MCE nucleoside, a 2'-O-MOE nucleoside, a 2'-O-NMA nucleoside, and a 2'-O-Me nucleoside.

15. The compound or a pharmaceutically acceptable salt thereof according to claim 14, wherein the 2'-modified nucleoside is at least one selected from the group consisting of a 2'-O-MCE nucleoside and a 2'-O-MOE nucleoside.

16. The compound or a pharmaceutically acceptable salt thereof according to any one of claims 1 to 15, wherein the modified oligonucleotide contains 5-methylcytosine.

17. The compound or a pharmaceutically acceptable salt thereof according to any one of claims 1 to 16,
wherein the modified oligonucleotide includes a gap segment, a 5' wing segment, and a 3' wing segment,
the gap segment contains at least 2 deoxyribonucleosides, and the 5' terminal and the 3' terminal of the gap segment are deoxyribonucleosides,
the nucleoside at the 3' terminal of the 5' wing segment is a sugar-modified nucleoside and is linked to the 5' terminal of the gap segment, and
the nucleoside at the 5' terminal of the 3' wing segment is a sugar-modified nucleoside and is linked to the 3' terminal of the gap segment.

18. The compound or a pharmaceutically acceptable salt thereof according to claim 17,
wherein the gap segment consists of 5 to 30 deoxyribonucleosides,
the 5' wing segment and the 3' wing segment each independently consist of 1 to 10 sugar-modified nucleosides independently selected from the group consisting of an LNA, a 2'-O-MCE nucleoside, and a 2'-O-MOE nucleoside, each of the wing segments containing at least one phosphorothioate bond, and
each cytosine in the gap segment and each wing segments is replaced with 5-methylcytosine.

19. The compound or a pharmaceutically acceptable salt thereof according to claim 18,
wherein the gap segment consists of 8 to 12 deoxyribonucleosides,
the 5' wing segment and the 3' wing segment each independently consist of 2 to 5 sugar-modified nucleosides independently selected from the group consisting of an LNA and a 2'-O-MCE nucleoside, and
the gap segment contains at least one phosphorothioate bond.

20. The compound according to claim 19, wherein the 5' wing segment and the 3' wing segment are each independently selected from the group consisting of LL, LLL, VLL, LVL, LLV, LVV, VLV, VVL, VVLL, VLVL, LVLV, LLVV, VLLL, LVLL, LLVL, LLLV, LVVV, VLVV, VVLV, and VVVL, where L represents an LNA, and V represents a 2'-O-MCE nucleoside.

21. The compound or a pharmaceutically acceptable salt thereof according to any one of claims 1 to 20, wherein the modified oligonucleotide is 11 to 50, preferably, 15 to 25 consecutive nucleosides long.

22. The compound or a pharmaceutically acceptable salt thereof according to any one of claims 1 to 21, wherein the modified oligonucleotide is an antisense oligonucleotide.

23. The compound or a pharmaceutically acceptable salt thereof according to any one of claims 1 to 22, wherein the compound containing the modified oligonucleotide contains a prodrug moiety.

24. The compound or a pharmaceutically acceptable salt thereof according to any one of claims 1 to 23, wherein the compound containing the modified oligonucleotide contains a functional molecule.

25. The compound or a pharmaceutically acceptable salt thereof according to claim 24, wherein the functional molecule is a group derived from a molecule having a function of delivering the modified oligonucleotide to a target site.

26. The compound or a pharmaceutically acceptable salt thereof according to claim 24 or 25, wherein the functional molecule is selected from the group consisting of a sugar, a lipid, a peptide, a protein, and a derivative thereof.

27. The compound or a pharmaceutically acceptable salt thereof according to any one of claims 24 to 26, wherein the functional molecule is a lipid selected from the group consisting of cholesterol, a vitamin, a steroid, a C5-30 saturated fatty acid, and a C5-30 unsaturated fatty acid.

28. The compound or a pharmaceutically acceptable salt thereof according to any one of claims 1 to 23, wherein the compound consists of the modified oligonucleotide.

29. The salt according to any one of claims 1 to 28, wherein the pharmaceutically acceptable salt is a sodium salt.

30. A drug comprising: the compound or a pharmaceutically acceptable salt according to any one of claims 1 to 29.

31. A drug for treating, preventing, and/or ameliorating a disease or a condition against which an inhibitory action on CALM2 gene expression is effective, the drug comprising:
the compound or a pharmaceutically acceptable salt according to any one of claims 1 to 29.

32. An inhibitor of CALM2 gene expression comprising:
the compound or a pharmaceutically acceptable salt according to any one of claims 1 to 29.

33. A drug for treating, preventing, and/or ameliorating congenital long QT syndrome, the drug comprising:
the compound or a pharmaceutically acceptable salt according to any one of claims 1 to 29.

34. A drug for treating, preventing, and/or ameliorating calmodulinopathy, the drug comprising: the compound or a pharmaceutically acceptable salt according to any one of claims 1 to 29.

35. A method for treating, preventing, and/or ameliorating a disease or a condition against which an inhibitory action on CALM2 gene expression is effective, the method comprising: a step of administering an effective dose of the compound or a pharmaceutically acceptable salt according to any one of claims 1 to 29 to a subject in need of the compound or a pharmaceutically acceptable salt.

36. A method for inhibiting CALM2 gene expression, the method comprising: a step of administering an effective dose of the compound or a pharmaceutically acceptable salt according to any one of claims 1 to 29 to a subject in need of the compound or a pharmaceutically acceptable salt.

37. A method for treating, preventing, and/or ameliorating congenital long QT syndrome, the method comprising: a step of administering an effective dose of the compound or a pharmaceutically acceptable salt according to any one of claims 1 to 29 to a subject in need of the compound or a pharmaceutically acceptable salt.

38. A method for treating, preventing, and/or ameliorating calmodulinopathy, the method comprising: a step of administering an effective dose of the compound or a pharmaceutically acceptable salt according to any one of claims 1 to 29 to a subject in need of the compound or a pharmaceutically acceptable salt.

39. The compound according to any one of claims 1 to 29, which is used as a drug.

40. The compound according to any one of claims 1 to 29, which is used for treating, preventing, and/or ameliorating a disease or a condition against which an inhibitory action on CALM2 gene expression is effective.

41. The compound according to any one of claims 1 to 29, which is used for inhibiting CALM2 gene expression.

42. The compound according to any one of claims 1 to 29, which is used for treating, preventing, and/or ameliorating congenital long QT syndrome.

43. The compound according to any one of claims 1 to 29, which is used for treating, preventing, and/or ameliorating calmodulinopathy.

44. Use of the compound or a pharmaceutically acceptable salt according to any one of claims 1 to 29 in production of a drug for treating, preventing, and/or ameliorating a disease or a condition against which an inhibitory action on CALM2 gene expression is effective.

45. Use of the compound or a pharmaceutically acceptable salt according to any one of claims 1 to 29 in production of an inhibitor of CALM2 gene expression.

46. Use of the compound or a pharmaceutically acceptable salt according to any one of claims 1 to 29 in production of a drug for treating, preventing, and/or ameliorating congenital long QT syndrome.

47. Use of the compound or a pharmaceutically acceptable salt according to any one of claims 1 to 29 in production of a drug for treating, preventing, and/or ameliorating calmodulinopathy.
